# EUROPEAN PATENT APPLICATION

(11) **EP 4 183 878 A1**
(43) Date of publication of application: **24.05.2023**
(21) Application number: 21842025.5
(22) Date of filing: 18.02.2021
(51) Int. Cl.: C12N 11/084

(54) **PVA MEMBRANE IMMOBILIZED ENZYME AND PREPARATION METHOD THEREFOR**

(30) Priority: 16.07.2020 CN 202010683476
(71) Applicant: Asymchem Life Science (Tianjin) Co., Ltd., Tianjin 300457 (CN)
(72) Inventor: HONG, Hao, Morrisville North Carolina 27560 (US); JAMES, , Gage, Morrisville North Carolina 27560 (US); XIAO, Yi, Tianjin 300457 (CN); ZHANG, Na, Tianjin 300457 (CN); VYASA, Williams, Morrisville North Carolina 27560 (US); CUI, Yuxia, Tianjin 300457 (CN); ZHAO, Jiadong, Tianjin 300457 (CN); GAO, Yanyan, Tianjin 300457 (CN)
(74) Representative: Delbarba, Andrea
(86) International application number: PCT/CN2021/076756
(87) International publication number: WO 2022/012041

(57) **Abstract**

Provided are a PVA membrane immobilized enzyme and a preparation method therefor. The PVA membrane immobilized enzyme comprises a PVA porous membrane and an enzyme entrapped on the PVA porous membrane. The PVA porous membrane is a three-dimensional structured PVA porous membrane. The enzyme is any one selected from transaminase, D-lactate dehydrogenase, cyclohexanone monooxygenase, ketoreductase, alkene reductase, nitrilase, ammonia lyase, amino acid dehydrogenase, imine reductase, alcohol dehydrogenase, ammonium formate dehydrogenase, glucose 1-dehydrogenase and mutants thereof. The three-dimensional structured PVA porous membrane is used as a carrier to immobilize an enzyme in an entrapment manner. The entrapment and immobilization process is simple, conditions are mild, and the specific surface area is large. After entrapping and immobilizing the enzyme in the PVA porous membrane, the enzyme is stable, and cannot be easily leached out in the process of use. The porous structure of the PVA porous membrane used can better transmit reactants and products, is suitable for use in continuous flow biochemical catalysis, and has wide applicability to enzymes.

## Description

### Technical Field

The prevent disclosure relates to the field of enzyme immobilization technologies, and specifically, to a PVA membrane immobilized enzyme and a preparation method therefor.

### Background

Biocatalysis is becoming an integral part of preparation programs for chemicals, intermediates, fine chemicals, and ultimately pharmaceutical molecules. However, with the increasing expansion of process requirements, the efficiency and economics of enzyme applications become inevitable. Therefore, it is required not only to improveenzyme activity, specificity and productivity, but also to prolong shelf life and recyclability, especially to facilitate the economic viability for commercial-scale applications.

An enzyme immobilization platform provides an excellent tool for properly integrating enzymes during production. Over the years, the enzyme immobilization efficiency of several natural and synthetic carrier pairs has been evaluated. For example, each platform performs special evaluation according to applications, economics, and advantages thereof. An immobilized biocatalyst is widely applied in the fields such as organic synthesis, pollution control and diagnosis (Enzyme Microb Technol, 31,171-8; J Pharm Sci, 89,979-90).

Immobilization is implemented by immobilizing an enzyme to a solid carrier or in the solid carrier, so as to obtain a heterogeneous immobilized enzyme system. The enzyme may be immobilized by means of a plurality of methods, including a physical method (there is a weak interaction between the carrier and the enzyme) and a chemical method (the carrier and the enzyme form a covalent bond) (Analyst, 133,697-701; Chem Soc Rev, 40,2567-92; Heidelberg, Berlin: Springer, 95-126.), or a combination of the physical method and the chemical method, so that carriers with various functional activities are included.

A method for physically immobilizing an enzyme includes adsorption (physical, ionic) in a membrane or a membrane reactor or on a water-insoluble substrate, for example, adsorption on a mesoporous material, inclusion (or gel entrapment), microencapsulation with a solid membrane, microencapsulation with a liquid membrane, formation of an enzymatic Langmuir-Blodgett membrane (Anal Chem, 1994; 66, 1120A-7A), and the like. When the membrane is entrapped or encapsulated, the obtained enzyme catalyst depends on the properties of a membrane support, such as hydrophilicity, hydrophobicity, density of a reactive functional group, porosity, pore size distribution, a membrane thickness, reactor configuration, and the like. The immobilization method is based on the positioning of the enzyme within the membrane, of which purpose is to achieve higher expression of the enzyme in addition to being highly stable under an operating condition.

At present, there are many studies on the immobilization of enzymes using porous membranes. However, since different enzymes have different structures and active sites, the immobilization methods thereof are also different. For example, when lipase is immobilized by Polyvinyl Alcohol membrane (PVA membrane), glutaraldehyde is required to be used for cross linking, so that the purpose of improving the stability and activity of the lipase by means of immobilization can be achieved. In addition, when a porous membrane is used to immobilize enzymes such as xylanase, catalase, cellulase, β-galactosidase and ascorbate oxidase, amino, carboxyl, sulfhydryl, hydroxyl, imidazole or phenolic groups are usually used to bond the porous membrane and the enzymes by means of covalent bonds. Therefore, a high-purity enzyme is required to be used when a crosslinking agent is used to immobilize the enzyme in the prior art, resulting in complex immobilization method and limited loading amount of the enzyme.

### Summary

The present disclosure is mainly intended to provide a PVA membrane immobilized enzyme and a preparation method therefor, to resolve a complex process of immobilizing an enzyme by porous membrane in the prior art.

In order to implement the above objective, an aspect of the present disclosure provides a PVA membrane immobilized enzyme. The PVA membrane immobilized enzyme includes a PVA porous membrane and an enzyme entrapped on the PVA porous membrane. The PVA porous membrane is a three-dimensional structured PVA porous membrane. The enzyme is any one selected from transaminase, D-lactate dehydrogenase, cyclohexanone monooxygenase, ketoreductase, alkene reductase, nitrilase, ammonia lyase, amino acid dehydrogenase, imine reductase, alcohol dehydrogenase, ammonium formate dehydrogenase, glucose 1 -dehydrogenase and mutants thereof.

Further, the enzyme is a free enzyme or a cross-linked enzyme aggregate.

Further, the transaminase is a transaminase derived from *ChromobacteriumviolaceumDSM30191,* or a transaminase derived from *Arthrobacter citreus,* or a transaminase derived from *B.thuringiensis;* the ketoreductase is a ketoreductase derived from *Acetobacter sp. CCTCC M209061* or a ketoreductase derived from *Candida macedoniensis AKU4588*; the cyclohexanone monooxygenase is a cyclohexanone monooxygenase derived from *Rhodococcus sp. Phil,* or a cyclohexanone monooxygenase derived from *Brachymonaspetroleovorans,* or a cyclohexanone monooxygenase derived from *Rhodococcus ruber-SD1;* the ammonia lyase is an ammonia lyase derived from *Aspergillus niger CBS 513.88or* an ammonia lyase derived from *Solenostemonscutellarioides;* the alkene reductase is an alkene reductase derived from *Saccharomyces cerevisiae* or an alkene reductase derived from *Chryseobacterium sp. CA49;* the imine reductase is an imine reductase derived from *Streptomyces* spot an imine reductase derived from *Bacillus cereus;* the amino acid dehydrogenase is an amino acid dehydrogenase derived from *Bacillus cereusor* an amino acid dehydrogenase derived from *Bacillus sphaericus;* the nitrilase is a nitrilase derived from *Aspergillus niger CBS 513.88or* anitrilase derived from *Neurospora crassa OR74A.*

Further, the transaminase derived from *Chromobacteriumviolaceum DSM30191* has an amino acid sequence shown in SEQ ID NO.1, and an amino acid sequence of a mutant of the transaminase is an amino acid sequence that is obtained by the mutation of the amino acid sequence shown in SEQ ID NO.1, wherein the mutation comprises at least one of the following mutation sites: 7th site, 47th site, 90th site, 95th site, 297th site, 304th site, 380th site, 405th site or 416th site, and threonine at the 7th site is mutated to cysteine, serine at the 47th site is mutated to the cysteine, lysine at the 90th site is mutated to glycine, alanine at the 95th site is mutated to proline, isoleucine at the 297th site is mutated to leucine, the lysine at the 304th site is mutated to aspartic acid, glutamine at the 380th site is mutated to the leucine, arginine at the 405th site is mutated to the glutamate, and the arginine at the 416th site is mutated to threonine, or the amino acid sequence of the mutant of the transaminase has the mutation site in the amino acid sequence that is obtained by means of mutation, and is of more than 80% identity with the amino acid sequence that is obtained by means of mutation.

Further, the transaminase derived from *Arthrobacter citreus* has an amino acid sequence shown in SEQ ID NO.2, and an amino acid sequence of a mutant of the transaminase is an amino acid sequence that is obtained by a mutation of the amino acid sequence shown in SEQ ID NO.2, wherein the mutation comprises at least one of the following mutation sites: 3rd site, 5th site, 60th site, 164th site, 171 st site, 178th site, 180th site, 186th site, 187th site, 252nd site, 370th site, 384th site, 389th site, 404th site, 411th site, 423rd site, or 424th site, and the leucine at the 3rd site is mutated to the serine, valine at the 5th site is mutated to the serine, the cysteine at the 60th site is mutated to tyrosine, phenylalanine at the 164th site is mutated to the leucine, the glutamate at the 171 st site is mutated to the aspartic acid, the alanine at the 178th site is mutated to the leucine, the isoleucine at the 180th site is mutated to the valine, the serine at the 186th site is mutated to glycine, the serine at the 187th site is mutated to the alanine, the valine at the 252nd site is mutated to the isoleucine, the leucine at the 370th site is mutated to the alanine, tyrosine at the 384th site is mutated to the phenylalanine, the isoleucine at the 389th site is mutated to the phenylalanine, the leucine at the 404th site is mutated to the glutamine, the glycine at the 411th site is mutated to the aspartic acid, methionine at the 423rd site is mutated to the lysine, and the glutamate at the 424th site is mutated to the glutamine, or the amino acid sequence of the mutant of the transaminase has the mutation site in the amino acid sequence that is obtained by means of mutation, and is of more than 80% identity with the amino acid sequence that is obtained by means of mutation.

Further, the ketoreductase derived from *Acetobacter sp. CCTCC M209061* has an amino acid sequence shown in SEQ ID NO.3, and an amino acid sequence of a mutant of the ketoreductase is an amino acid sequence that is obtained by a mutation of the amino acid sequence shown in SEQ ID NO.3, wherein the mutation comprises at least one of the following mutation sites: 94th site, 144th site, or 156th site, and the alanine at the 94th site is mutated to asparagine, the glutamate at the 144th site is mutated to the serine, and the asparagine at the 156th site is mutated to the threonine or the valine, or the amino acid sequence of the mutant of the ketoreductase has the mutation site in the amino acid sequence that is obtained by means of mutation, and is of more than 80% identity with the amino acid sequence that is obtained by means of mutation.

Further, the cyclohexanone monooxygenase derived *fromRhodococcus sp. Phil* has an amino acid sequence shown in SEQ ID NO.4, and an amino acid sequence of a mutant of the cyclohexanone monooxygenase is an amino acid sequence that is obtained by a mutation of the amino acid sequence shown in SEQ ID NO.4, wherein the mutation comprises at least one of the following mutation sites: 280th site, 435th site, 436th site, 438th site, 411 st site, 508th site, or 510th site, and the phenylalanine at the 280th site is mutated to the tyrosine, the phenylalanine at the 435th site is mutated to the asparagine, the phenylalanine at the 436th site is mutated to the serine, the leucine at the 438th site is mutated to the alanine, serine at the 411st site is mutated to the valine, and the leucine at the 510th site is mutated to the valine, or the amino acid sequence of the mutant of the cyclohexanone monooxygenase has the mutation site in the amino acid sequence that is obtained by means of mutation, and is of more than 80% identity with the amino acid sequence that is obtained by means of mutation.

Further, the cyclohexanone monooxygenase derived from *Rhodococcus ruber-SD1* has an amino acid sequence shown in SEQ ID NO.5, and an amino acid sequence of the mutant of the cyclohexanone monooxygenase is an amino acid sequence that is obtained by a mutation of the amino acid sequence shown in SEQ ID NO.5, wherein the mutation comprises at least one of the following mutation sites: 45th site, 190th site, 249th site, 257th site, 393rd site, 504th site, or 559th site, and methionine at the 45th site is mutated to the threonine, proline at the 190th site is mutated to the leucine, the cysteine at the 249th site is mutated to the valine, the cysteine at the 257th site is mutated is the alanine, the cysteine at the 393rd site is mutated to the valine, the proline at the 504th site is mutated to the valine, and the tyrosine at the 559th site is mutated to the methionine, or the amino acid sequence of the mutant of the cyclohexanone monooxygenase has the mutation site in the amino acid sequence that is obtained by means of mutation, and is of more than 80% identity with the amino acid sequence that is obtained by means of mutation.

Further, the PVA membrane immobilized enzyme further includes a coenzyme and a cofactor of each enzyme, and the coenzyme and the cofactor are entrapped on the PVA porous membrane.

Further, the PVA porous membrane further has polyethylene glycol and/or polyethyleneimine. A molecular weight of the polyethylene glycol is PEG400-PEG 6000, and a molecular weight of the polyethyleneimine is 3KDa-70KDa.

Further, a mass ratio of the polyethylene glycol to the porous PVA is 5:4-75:4, and a mass ratio of the polyethyleneimine to the porous PVA is 1:12-1:240.

Further, the enzyme is a crude enzyme.

Further, the loading amount of the enzyme is 0.05 ~ 0.4 g of free enzyme/cm² membrane or 0.03 ~ 0.06 g of dry cross-linked enzyme aggregate/cm² membrane.

Another aspect of the present disclosure provides a method for preparing the PVA membrane immobilized enzyme described in any one of the above. The preparation method includes: S1, mixing a raw material comprising an enzyme an a PVA solution for scheduled time, to obtain a mixed system; S2, adding the mixed system to a mold, and drying the mixed system to obtain a membrane-entrapped enzyme, wherein the mold is a three-dimensional structured mold so as to form a three-dimensional structured PVA porous membrane; and S3, using a phosphate buffer solution to soak and wash the membrane-entrapped enzyme, and then obtaining the PVA membrane immobilized enzyme.

Further, a pH value of the mixed system is 6.0-6.5.

Further, S1 includes: preparing suspension liquid or an enzyme solution of the enzyme, where the enzyme in the suspension liquid is a cross-linked enzyme aggregate, and the enzyme in the enzyme solution is a free enzyme without cells; and mixing the suspension liquid or the enzyme solution with the PVA solution for the scheduled time, to obtain the mixed system.

Further, the scheduled time is 10-60 min. A PVA molecular weight of the PVA solution is 20KDa-200KDa.

Further, the content of PVA in the PVA solution is 10-50 g/100 mL.

Further, acetic acid, methanol and sulfuric acid are dispersed in the PVA solution.

Further, a pH value of the PVA solution is 5.5-6.5.

Further, a ratio of the enzyme to the PVA solution is 1-50 g/100 mL.

Further, the suspension liquid or the enzyme solution further contains the phosphate buffer solution, an optional cofactor and an optional coenzyme.

Further, a weight ratio of the coenzyme to the enzyme is 10:1-1:10.

Further, S1 includes: mixing a PVA aqueous solution and cross-linked enzyme particles to form the mixed system.

Further, a ratio of the cross-linked enzyme particle to the PVA aqueous solution is 1-50 g/100 mL.

Further, the scheduled time is 10-60 min.

Further, the cross-linked enzyme particle includes the enzyme, an optional cofactor and an optional coenzyme.

Further, S1 includes: mixing a PVA aqueous solution and a modifier solution for a first scheduled time, to form a second mixed system; and mixing the second mixed system and an enzyme system for a second scheduled time, to form the mixed system.

Further, a concentration of the PVA aqueous solution is 5-30 g/100 mL. Preferably, the modifier solution includes a polyethylene glycol aqueous solution in which the cofactors are dispersed and/or a polyethyleneimine aqueous solution in which the cofactors are dispersed.

Further, a molecular weight of the polyethylene glycol is PEG400-PEG6000, and a concentration of the polyethylene glycol in the mixed system is 3-10 g/100 mL.

Further, a molecular weight of the polyethyleneimine is 3KDa-70KDa, and more preferably, 3KDa-50KDa.

Further, a concentration of the polyethyleneimine in the mixed system is 0.1-1 g/100 mL, and more preferably, 0.1-0.3 g/100 mL.

Further, the enzyme system includes the enzyme, the optional cofactor, the optional coenzymeand the phosphate buffer solution.

Further, the enzyme is a free enzyme or a cross-linked enzyme aggregate without cells.

Further, a concentration of the cofactor in the enzyme system is 1-20 mg/mL.

Further, a weight ratio of the coenzyme in the enzyme system to the enzyme is 10:1-1:10.

Further, a ratio of the enzyme to the PVA solution is 1-50 g/100 mL.

Further, S2 includes: placing the mixed system in a mold for a third scheduled time, and then adding a dehydration accelerator to the mold for drying. The dehydration accelerator is any one or more selected from a group consisting of acetonitrile, ethanol and acetone.

Further, a volume ratio of the dehydration accelerator to the mixed system is 1 :10-5:1.

Further, the third scheduled time is 2-4 h.

Further, the mold is the three-dimensional structured mold, and the three-dimensional structured mold has protrusions or grooves.

Further, S3 includes: soaking the membrane-entrapped enzyme in the phosphate buffer solution for 2-16 h, and then using the fresh phosphate buffer solution to wash the membrane-entrapped enzyme, so as to obtain the PVA membrane immobilized enzyme.

Through the application of the technical solution of the prevent disclosure, by means of using the PVA porous membrane as the carrier to immobilize the enzyme in an entrapment manner, the entrapment and immobilization process is simple, conditions are mild, and desirable immobilization effect is achieved on either purified enzymes or crude enzymes. After entrapping and immobilizing the enzyme in the PVA porous membrane, the enzyme is stable, and cannot be easily leached out during use. The porous structure of the PVA porous membrane used can better transmit reactants and products, and is suitable for use in continuous flow biochemical catalysis. Since entrapment and immobilization described above is mechanical immobilization, the porous membrane has wide applicability to enzymes. As the three-dimensional structured PVA porous membrane has the three-dimensional structure, the PVA porous membrane may have more specific surface areas, so that more entrapment sites can be provided. Therefore, the loading amount of the enzymes is increasedon the basis of guaranteeing of high activity and stability of the enzymes.

### Brief Description of the Drawings

The drawings, which form a part of this disclosure, are used to provide a further understanding of the present disclosure. The exemplary embodiments of the present disclosure and the description thereof are used to explain the present disclosure, but do not constitute improper limitations to the present disclosure. In the drawings:
Fig. 1 is a stability curve of a PVA membrane immobilized enzyme with and without PLP according to Embodiment 2 of the present disclosure.
Fig. 2 is a stability curve of two PVA membrane immobilized enzymes according to Embodiment 3 of the present disclosure.
Fig. 3 is a stability curve of a PVA membrane immobilized enzyme with and without PLP according to Embodiment 5 of the present disclosure.
Fig. 4 is a stability curve of a PVA membrane immobilized enzyme with and without PLP according to Embodiment 6 of the present disclosure.
Fig. 5 is a stability curve of a PVA membrane immobilized enzyme with PLP and with NAD⁺ according to Embodiment 8 of the present disclosure.
Fig. 6 is a stability curve of a PVA membrane immobilized enzyme with PLP and with NAD⁺ according to Embodiment 8 of the present disclosure.
Fig. 7 is a stability curve of a PVA membrane immobilized enzyme with and without PLP according to Embodiment 9 of the present disclosure.

### Detailed Description of the Embodiments

It is to be noted that the embodiments in this disclosure and the features in the embodiments may be combined with one another without conflict. The disclosure will be described below in detail with reference to the drawings and the embodiments.

As analyzed in the Background of this disclosure, the process of immobilizing an enzyme using a porous membrane in the prior art is complicated. In order to resolve this problem, this disclosure provides a PVA membrane immobilized enzyme and a preparation method therefor.

A typical implementation of this disclosure provides a PVA membrane immobilized enzyme. The PVA membrane immobilized enzyme includes a PVA porous membrane and an enzyme entrapped on the PVA porous membrane. The PVA porous membrane is a three-dimensional structured PVA porous membrane. The enzyme is any one selected from transaminase (for example, ω-transaminase), D-lactate dehydrogenase, formate dehydrogenase, carbonyl reductase, cyclohexanone monooxygenase, ketene reductase, nitrilase, ammonia lyase, amino acid dehydrogenase, imine reductase, and mutants thereof.

In this disclosure, by means of using the PVA porous membrane as the carrier to immobilize the enzyme in an entrapment manner, the entrapment and immobilization process is simple, conditions are mild, and desirable immobilization effect is achieved on either purified enzymes or crude enzymes. After entrapping and immobilizing the enzyme in the PVA porous membrane, the immobilization of the enzyme is more stable than that in a planar PVA membrane, and cannot be easily leached out during use. The porous structure of the PVA porous membrane used can better transmit reactants and products, and is suitable for use in continuous flow biochemical catalysis. Since entrapment and immobilization described above is mechanical immobilization, the porous membrane has wide applicability to enzymes. As the three-dimensional structured PVA porous membrane has the three-dimensional structure, the PVA porous membrane may have more specific surface areas, so that more entrapment sites can be provided. Therefore, the loading amount of the enzymes is increasedon the basis of guaranteeing of high activity and stability of the enzymes.

The enzyme entrapped in the PVA membrane immobilized enzyme may be a free enzyme or a cross-linked enzyme aggregate. Catalytic effects of both the free enzyme and the cross-linked enzyme aggregate may be effectively achieved after entrapment.

The cross-linked enzyme aggregate is similar to that in the prior art, which is an insoluble enzyme aggregate that is obtained by using ammonium sulphate or a precipitation agent such as ethanol, acetonitrile, acetone, propanol and PEG to precipitate the free enzyme, and then adding a bifunctional reagent such as glutaraldehyde, glyoxal or aldehyde dextranfor covalent cross-linking.

As described above, the PVA membrane immobilized enzyme has wide applicability to enzymes, which is more suitable for the following enzymes. The transaminase is a transaminase derived from *Chromobacteriumviolaceum DSM30191,* or a transaminase derived from *Arthrobacter citreus,* or a transaminase derived from *B.thuringiensis.* The ketoreductase is a ketoreductase derived from *Acetobacter sp. CCTCC M209061* or a ketoreductase derived from *Candida macedoniensis AKU4588.* The cyclohexanone monooxygenase is a cyclohexanone monooxygenase derived from *Rhodococcussp. Phil,* or a cyclohexanone monooxygenase derived *fromBrachymonaspetroleovorans,* or a cyclohexanone monooxygenase derived from *Rhodococcus ruber-SD1.* The ammonia lyase is an ammonia lyase derived from *Aspergillus niger CBS 513.88* and an ammonia lyase derived from *Solenostemonscutellarioides.* The alkene reductase is an alkene reductase derived from *Saccharomyces cerevisiae* and an alkene reductase derived *fromChryseobacteriumsp. CA49.* The imine reductase is an imine reductase derived from *Streptomyces sp* and an imine reductase derived from *Bacillus cereus.* The amino acid dehydrogenase is an amino acid dehydrogenase derived from *Bacillus cereus* and an amino acid dehydrogenase derived from *Bacillus sphaericus.* The nitrilase is a nitrilase derived from *Aspergillus niger CBS 513.88* and a nitrilase derived from *Neurospora crassa OR74A.* Preferably, the transaminase derived from *Chromobacteriumviolaceum DSM30191* has an amino acid sequence shown in SEQ ID NO.1, and an amino acid sequence of a mutant of the transaminase is an amino acid sequence that is obtained by mutating the amino acid sequence shown in SEQ ID NO.1. The mutation includes at least one of the following mutation sites: 7th site, 47th site, 90th site, 95th site, 297th site, 304th site, 380th site, 405th site or 416th site. Threonine at the 7th site is mutated to cysteine, serine at the 47th site is mutated to the cysteine, lysine at the 90th site is mutated to glycine, alanine at the 95th site is mutated to proline, isoleucine at the 297th site is mutated to leucine, the lysine at the 304th site is mutated to aspartic acid, glutamine at the 380th site is mutated to the leucine, arginine at the 405th site is mutated to the glutamate, and the arginine at the 416th site is mutated to threonine; or the amino acid sequence of the mutant of the transaminase has the mutation site in the amino acid sequence that is obtained by means of mutation, and is of more than 80% identity with the amino acid sequence that is obtained by means of mutation. Preferably, the transaminase derived from *Arthrobacter citreus* has an amino acid sequence shown in SEQ ID NO.2, and an amino acid sequence of a mutant of the transaminase is an amino acid sequence that is obtained by mutating the amino acid sequence shown in SEQ ID NO.2. The mutation includes at least one of the following mutation sites: 3rd site, 5th site, 60th site, 164th site, 171st site, 178th site, 180th site, 186th site, 187th site, 252nd site, 370th site, 384th site, 389th site, 404th site, 411th site, 423rd site, or 424th site. The leucine at the 3rd site is mutated to the serine, valine at the 5th site is mutated to the serine, the cysteine at the 60th site is mutated to tyrosine, phenylalanine at the 164th site is mutated to the leucine, the glutamate at the 171st site is mutated to the aspartic acid, the alanine at the 178th site is mutated to the leucine, the isoleucine at the 180th site is mutated to the valine, the serine at the 186th site is mutated to glycine, the serine at the 187th site is mutated to the alanine, the valine at the 252nd site is mutated to the isoleucine, the leucine at the 370th site is mutated to the alanine, tyrosine at the 384th site is mutated to the phenylalanine, the isoleucine at the 389th site is mutated to the phenylalanine, the leucine at the 404th site is mutated to the glutamine, the glycine at the 411th site is mutated to the aspartic acid, methionine at the 423rd site is mutated to the lysine, and the glutamate at the 424th site is mutated to the glutamine; or the amino acid sequence of the mutant of the transaminase has the mutation site in the amino acid sequence that is obtained by means of mutation, and is of more than 80% identity with the amino acid sequence that is obtained by means of mutation. Preferably, the ketoreductase derived from *Acetobacter sp. CCTCC M209061* has an amino acid sequence shown in SEQ ID NO.3, and an amino acid sequence of a mutant of the ketoreductase is an amino acid sequence that is obtained by mutating the amino acid sequence shown in SEQ ID NO.3. The mutation includes at least one of the following mutation sites: 94th site, 144th site, or 156th site. The alanine at the 94th site is mutated to asparagine, the glutamate at the 144th site is mutated to the serine, and the asparagine at the 156th site is mutated to the threonine or the valine; or the amino acid sequence of the mutant of the ketoreductase has the mutation site in the amino acid sequence that is obtained by means of mutation, and is of more than 80% identity with the amino acid sequence that is obtained by means of mutation. Preferably, the cyclohexanone monooxygenase derived from *Rhodococcussp. Phil* has an amino acid sequence shown in SEQ ID NO.4, and an amino acid sequence of a mutant of the cyclohexanone monooxygenase is an amino acid sequence that is obtained by mutating the amino acid sequence shown in SEQ ID NO.4. The mutation includes at least one of the following mutation sites: 280th site, 435th site, 436th site, 438th site, 411st site, 508th site, or 510th site. The phenylalanine at the 280th site is mutated to the tyrosine, the phenylalanine at the 435th site is mutated to the asparagine, the phenylalanine at the 436th site is mutated to the serine, the leucine at the 438th site is mutated to the alanine, serine at the 411st site is mutated to the valine, and the leucine at the 510th site is mutated to the valine; or the amino acid sequence of the mutant of the cyclohexanone monooxygenase has the mutation site in the amino acid sequence that is obtained by means of mutation, and is of more than 80% identity with the amino acid sequence that is obtained by means of mutation. Preferably, the cyclohexanone monooxygenase derived from *Rhodococcus ruber-SD1* has an amino acid sequence shown in SEQ ID NO.5, and an amino acid sequence of the mutant of the cyclohexanone monooxygenase is an amino acid sequence that is obtained by mutating the amino acid sequence shown in SEQ ID NO.5. The mutation includes at least one of the following mutation sites: 45th site, 190th site, 249th site, 257th site, 393rd site, 504th site, or 559th site. Methionine at the 45th site is mutated to the threonine, proline at the 190th site is mutated to the leucine, the cysteine at the 249th site is mutated to the valine, the cysteine at the 257th site is mutated is the alanine, the cysteine at the 393rd site is mutated to the valine, the proline at the 504th site is mutated to the valine, and the tyrosine at the 559th site is mutated to the methionine; or the amino acid sequence of the mutant of the cyclohexanone monooxygenase has the mutation site in the amino acid sequence that is obtained by means of mutation, and is of more than 80% identity with the amino acid sequence that is obtained by means of mutation.

In order to improvethe catalytic efficiency of the PVA membrane immobilized enzyme, and preferably, the PVA membrane immobilized enzyme further includes a coenzyme and a cofactor of each enzyme. The coenzyme and the cofactor of each enzyme mean that, if the enzyme has the corresponding coenzyme and the cofactor, the coenzyme and the cofactor of the enzyme are also entrapped in the PVA membrane immobilized enzyme; and if the enzyme does not have the coenzyme and the cofactor, excess coenzymes or cofactors are not arranged in the PVA membrane immobilized enzyme.

The PVA membrane immobilized enzyme in this disclosure may be suitable for either purified enzymes or crude enzymes. In order to simplify processes, the enzyme is preferably the crude enzyme. In addition, since the immobilization mode of this disclosure is entrapment, more enzymes can be loaded. Preferably, the loading amount of the enzyme is 0.05 ~ 0.4 g of free enzyme/cm² membrane or 0.03 ~ 0.06 g of dry cross-linked enzyme aggregate/cm² membrane.

In another embodiment, the PVA porous membrane further has polyethylene glycol and/or polyethyleneimine. A molecular weight of the polyethylene glycol is PEG400-PEG 6000, and a molecular weight of the polyethyleneimine is 3KDa-70KDa, preferably, 3KDa-50KDa. Further, preferably, a mass ratio of the polyethylene glycol to the porous PVA is 5:4-75:4, and a mass ratio of the polyethyleneimine to the porous PVA is 1:12-1:240. By polyethylene glycol and the polyethyleneimine, a pore structure in the PVA porous membrane can be more abundant.

Another typical implementation of this disclosure provides a method for preparing the PVA membrane immobilized enzyme described in any one of the above. The preparation method includes: S1, mixing a raw material comprising an enzyme an a PVA solution for scheduled time, to obtain a mixed system; S2, adding the mixed system to a mold, and drying the mixed system to obtain a membrane-entrapped enzyme, wherein the mold is a three-dimensional structured mold so as to form a three-dimensional structured PVA porous membrane; and S3, using a phosphate buffer solution to soak and wash the membrane-entrapped enzyme, and then obtaining the PVA membrane immobilized enzyme. Preferably, a pH value of the mixed system is 6.0-6.5.

The preparation method in this disclosure may form the PVA membrane immobilized enzyme by means of mixing, drying and post-processing, so that the preparation method is simple in process and easy to operate, and does not need to use glutaraldehyde, amino and carboxyl for cross-linking or covalent immobilization. The PVA porous membrane in the formed PVA membrane immobilized enzyme is used as the carrier to immobilize the enzyme in an entrapment manner, so that a desirable immobilization effect is achieved on either the purified enzymes or the crude enzymes. After entrapping and immobilizing the enzyme in the PVA porous membrane, the enzyme is stable, and cannot be easily leached out during use. The porous structure of the PVA porous membrane used can better transmit reactants and products, and is suitable for use in continuous flow biochemical catalysis. Since entrapment and immobilization described above is mechanical immobilization, the porous membrane has wide applicability to enzymes. By using a three-dimensional structured tool to perform three-dimensional structuring on the PVA porous membrane, the PVA porous membrane may have a three-dimensional structure, so that more specific surface areas are obtained, so as to provide more entrapment sites. Therefore, the loading amount of the enzymes is increasedon the basis of guaranteeing of high activity and stability of the enzymes.

The method for forming the mixed system may vary according to the form of the enzymes provided. Provided below are several optional methods for forming the mixed system, and the description of S1 cannot be regarded as limitations to the scope of S1.

In an embodiment of this disclosure, S1 includes: preparing suspension liquid or an enzyme solution of the enzyme, where the enzyme in the suspension liquid is a cross-linked enzyme aggregate, and the enzyme in the enzyme solution is a free enzyme without cells; and mixing the suspension liquid or the enzyme solution with the PVA solution for the scheduled time, to obtain the mixed system. Either the enzyme solution or the suspension liquid of the enzyme may be mixed with the PVA solution, and mechanical stirring or magnetic stirring may be performed during mixing.

The PVA solution is a mixed solution including PVA, water, acetic acid, methanol and sulfuric acid. Preferably, a pH value of the mixed solution is between 5.5 and 6.5. A prepared mode can have more micropores by combining the acetic acid, methanol and sulfuric acid.

In order to improve the uniformity of the enzyme dispersed in the PVA solution, preferably, the scheduled time is 2-4 h.

In order to form a gel membrane with more reliable mechanical strength, the PVA molecular weight of the PVA solution is 20KDa-200KDa. In addition, in order to further form the PVA porous membrane with abundant porosity so as to facilitate the dispersion of the enzyme therein, preferably, the content of PVA in the PVA solution is 5-30 g/100 mL, preferably, 10-50 g/100 mL.

On the basis of mechanical immobilization of the PVA membrane-entrapped enzyme, the loading amount of the enzyme may be relatively large. Preferably, a concentration of the enzyme in the suspension liquid or the enzyme solution is 0.1-0.5 g/mL. Preferably, the ratio of the enzyme to the PVA solution is 1-50 g/100 mL, more preferably, 5-40 g/100 mL.

During mixing, in order to maintain the high activity of the enzyme, the suspension liquid or the enzyme solution further includes the phosphate buffer solution, an optional cofactor and an optional coenzyme. Preferably, a concentration of the cofactor is 1-20 mg/mL. Preferably, a weight ratio of the coenzyme to the enzyme is 10:1-1 :10.

In another embodiment of this disclosure, S1 includes: mixing a PVA aqueous solution and cross-linked enzyme particles to form the mixed system. In this embodiment, the cross-linked enzyme is mixed with the PVA aqueous solution in the form of dry particles, so that the cross-linked enzyme particles are easy to disperse. In order to guarantee the loading amount of the enzyme, preferably, a ratio of the cross-linked enzyme particles to the PVA aqueous solution is 1-50 g/100 mL. Likewise, in order to improve the uniformity of the cross-linked enzyme particles dispersed in the PVA aqueous solution, preferably, the scheduled time is 10-60 min. In addition, if required, preferably, the cross-linked enzyme particle includes the enzyme, the optional cofactor and the optional coenzyme.

In still another embodiment of this disclosure, S1 includes: mixing the PVA aqueous solution and a modifier solution for a first scheduled time, to form a second mixed system; and mixing the second mixed system and an enzyme system for a second scheduled time, to form the mixed system. The modifier is used to facilitate the membrane formation of PVA and enrich the pore structures therein.

In order to further form the PVA porous membrane with abundant porosity so as to facilitate the dispersion of the enzyme therein, preferably, the concentration of the PVA aqueous solution is 5-30 g/100 mL. The modifier may be selected from modifiers that are commonly used during the membrane formation of PVA. In order to avoid the impact of the modifier on the enzyme, the modifier solution preferably includes a mixed solution in which the acetic acid, methanol and sulfuric acid are dispersed, and/or a polyethylene glycol aqueous solution in which the cofactors are dispersed, and/or a polyethyleneimine aqueous solution in which the cofactors are dispersed. Preferably, the content of the acetic acid in the mixed solution is 2-4 g/100 mL, the content of the methanol is 5-9 g/100 mL, and the content of the sulfuric acid is 0.5-1 g/100 mL. Preferably, a molecular weight of the polyethylene glycol is PEG400-PEG6000. Preferably, a concentration of the polyethylene glycol in the mixed system is 3-10 g/100 mL. Preferably, a molecular weight of the polyethyleneimine is 3KDa-70KDa, and more preferably, 3KDa-50KDa. Preferably, a concentration of the polyethyleneimine in the mixed system is 0.1-1 g/100 mL, and more preferably, 0.1-0.3 g/100 mL.

When the modifier solution is added, the mixing between the enzyme system and the second mixed system is not obviously affected. Therefore, the enzyme system may be the common system form for enzyme supply. Preferably, the enzyme system includes the enzyme, the optional cofactor, the optional coenzyme and the phosphate buffer solution. Preferably, the enzyme is a free enzyme or a cross-linked enzyme aggregate without cells. In order to increase the loading amount of the enzyme, when the cofactor and the coenzyme are required to be used, the concentration of the cofactor is 1-20 mg/mL. Preferably, a weight ratio of the coenzyme in the enzyme system to the enzyme is 10:1-1:10, and a ratio of the enzyme to the PVA solution is 1-50 g/100 mL.

After the mixed system is formed, the mixed system may be allowed to stand for drying. In order to accelerate a membrane-formation process, preferably, S2 includes: placing the mixed system in a mold for a third scheduled time, and then adding a dehydration accelerator to the mold for drying. The dehydration accelerator isanyone or more selected from a group consisting of acetonitrile, ethanol and acetone. Preferably, a volume ratio of the dehydration accelerator to the mixed system is 1:10-5:1. During membrane formation, the enzyme is entrapped by the formed PVA porous membrane, so as to form a stable immobilized enzyme structure. In order to prevent rapid membrane formation, causing the enzyme to not be completed coated, preferably, the third scheduled time is 2-4 h. In addition, in order to simplify the structure of the three-dimensional structured mold, the three-dimensional structured mold has protrusions or grooves, preferably.

After membrane formation, in order to cause the enzyme to be immobilized more firmly, preferably, S3 includes: soaking the membrane-entrapped enzyme in the phosphate buffer solution for 2-16 h, and then using the fresh phosphate buffer solution to wash the membrane-entrapped enzyme, so as to obtain the PVA membrane immobilized enzyme.

In the preparation method of this disclosure, the used enzyme may be the purified enzyme or the crude enzyme. In order to reduce cost, the enzyme is the crude enzyme, preferably.

The PVA membrane immobilized enzyme obtained in this disclosure may further be resuspended with a buffer solution, and then the glutaraldehyde is added for modification, to cause enzyme molecules to cross-link with each other by means of covalent bonding between amino and an aldehyde group of the glutaraldehyde, so as to form a larger aggregate, so that the enzyme does not leak from the PVA membrane. In addition, the enzyme attached to the surface layer of the PVA membrane may also be covalently linked to the PVA by means of the arm action of the glutaraldehyde, so that firmer immobilization can be achieved, and the number of use can be increased. Preferably, the use amount of the glutaraldehyde is 1-2 g/100 mL suspension liquid.

The beneficial effects of this disclosure are further described below with reference to the embodiments and comparative examples.

Enzymes used in the following embodiments and sources thereof are shown in Table 1.

**Table 1**

| Enzyme | Abbreviation | Species origin |
|---|---|---|
| Transaminase | TA-Cv | *Chromobacteriumviolaceum DSM 30191* |
| | TA-Ac | *Arthrobacter citreus* |
| | TA-Bt | *B. thuringiansis* |
| Ketoreductase | KRED-Ac | *Acetobacter sp. CCTCC M209061* |
| | KRED-Cm | *Candida macedoniensiz. AKU4588* |
| Alcohol dehydrogenase | ADH-Tb | *Thermoanaerobiumbrockii* |
| D-lactate dehydrogenase | D-LDH | *Lactobacillus helveticus* |
| Ammonium formate dehydrogenase | FDH | *Candida boidinii* |
| Glucose 1-dehydrogenase | GDH | *Lysinibacillussphaericus G10* |
| Cyclohexanone monooxygenase | CHMO-Rs | *Rhodococcussp. Phil* |
| | CHMO-Bp | *Brachymonaspetroleo vorans* |
| | CHMO-Rr | *Rhodococcus ruber-SD1* |
| Alkene reductase | ERED-Sc | *Saccharomyces cerevisiae* |
| | ERED-Chr | *Chryseobacteriumsp. CA49* |
| Imine reductase | IRED-Str | *Streptomyces sp.* |
| | IRED-Bc | *Bacillus cereus* |
| Amino acid dehydrogenase | AADH-Bc | *Bacillus cereus* |
| | AADH-Bs | *Bacillus sphaericus* |
| Ammonia lyase | PAL-An | *Aspergillus niger CBS 513.88* |
| | PAL-Ss | *Solenostemonscutellarioides* |
| Nitrilase | NIT-An | *Aspergillus niger CBS 513.88* |
| | NIT-Nc | *Neurospora crassa OR74A* |

An amino acid sequence of the TA-Cv transaminase is SEQ ID NO.1:

MQKQRTTSQWRELDAAHHLHPFTDTASLNQAGARVMTRGEGVYLWDSEGNKIIDGMAGLW
CVNVGYGRKDFAEAARRQMEELPFYNTFFKTTHPAVVELSSLLAEVTPAGFDRVFYTNSGSESVD
TMIRMVRRYWDVQGKPEKKTLIGRWNGYHGSTIGGASLGGMKYMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT

YSGHPVCAAVAHANVAALRDEGIVQRVKDDIGPYMQKRWRETFSRFEHVDDVRGVGMVQAFTLV
KNKAKRELFPDFGEIGTLCRDIFFRNNLIMRACGDHIVSAPPLVMTRAEVDEMLAVAERCLEEFEQT
LKARGLA.

A mutation site and amino acid mutation of a mutant 1(TA-Cv-V1) are: R416T+T7C+S47C+Q380L; and a mutation site and amino acid mutation of a mutant 2(TA-Cv-V2) are: R416T+T7C+S47C+R405E+K90G+A95P+K304D+Q380L+I297L.

An amino acid sequence of the TA-Ac transaminase is SEQ ID NO.2:

MGLTVQKINWEQVKEWDRKYLMRTFSTQNEYQPVPIESTEGDYLITPGGTRLLDFFNQLCCV
NLGQKNQKVNAAIKEALDRYGFVWDTYATDYKAKAAKIIIEDILGDEDWPGKVRFVSTGSEAVETAL
NIARLYTNRPLVVTREHDYHGWTGGAATVTRLRSFRSGLVGENSESFSAQIPGSSCSSAVLMAPS
SNTFQDSNGNYLKDENGELLSVKYTRRMIENYGPEQVAAVITEVSQGVGSTMPPYEYVPQIRKMT
KELGVLWISDEVLTGFGRTGKWFGYQHYGVQPDIITMGKGLSSSSLPAGAVVVSKEIAAFMDKHR
WESVSTYAGHPVAMAAVCANLEVMMEENLVEQAKNSGEYIRSKLELLQEKHKSIGNFDGYGLLWI
VDIVNAKTKTPYVKLDRNFRHGMNPNQIPTQIIMEKALEKGVLIGGAMPNTMRIGASLNVSRGDIDK
AMDALDYALDYLESGEWQQS.

A mutation site and amino acid mutation of a mutant 1(TA-Ac-V1) are: L3S+V5S+C60Y+F164L+A178L+S187A+I180V+L370A+G411D+S186G+Y384F+I389F+V252I+L4 04Q+E171D; and a mutation site and amino acid mutation of a mutant 2(TA-Ac-V2) are: L3S+V5S+C60Y+F164L+A178L+S187A+I180V+L370A+G411D+S186G+Y384F+I389F+V252I+E4 24Q+M423K.

An amino acid sequence of the KRED-Ac ketoreductase is SEQ ID NO.3:

MARVAGKVAIVSGAANGIGKATAQLLAKEGAKVVIGDLKEEDGQKAVAEIKAAGGEAAFVKLNV
TDEAAWKAAIGQTLKLYGRLDIAVNNAGINYSGSVESTSLEDWRRVQSINLDGVFLGTQVAIEAMK
KSGGGSIVNLSSISGLIGDPMLAAYVASKGGVRLFTKSAALHCAKSGYKIRVNSVHPGYIWTPMVA
GLTKEDAAARQKLVDLHPIGHLGEPNDIAYGILYLASDESKFVTGSELVIDGGYTAQ.

A mutation site and amino acid mutation of a mutant 1(KRED-Ac-V1) are: E144S+A94N+N156V; and a mutation site and amino acid mutation of a mutant 2(KRED-Ac-V2) are: E144S+A94T+N156T.

An amino acid sequence of the CHMO-Rs cyclohexanone monooxygenase is SEQ ID NO.4:

MTAQISPTVVDAVVIGAGFGGIYAVHKLHNEQGLTVVGFDKADGPGGTWYWNRYPGALSDTE
SHLYRFSFDRDLLQDGTWKTTYITQPEILEYLESVVDRFDLRRHFRFGTEVTSAIYLEDENLWEVST

DKGEVYRAKYVVNAVGLLSAINFPDLPGLDTFEGETIHTAAWPEGKNLAGKRVGVIGTGSTGQQVI
TALAPEVEHLTVFVRTPQYSVPVGNRPVTKEQIDAIKADYDGIWDSVKKSAVAFGFEESTLPAMSVS
EEERNRIFQEAWDHGGGFRFMFGTFGDIATDEAANEAAASFIRSKIAEIIEDPETARKLMPTGLYAK
RPLCDNGYYEVYNRPNVEAVAIKENPIREVTAKGVVTEDGVLHELDVLVFATGFDAVDGNYRRIEIR
GRNGLHINDHWDGQPTSYLGVTTANFPNWFMVLGPNGPFTNLPPSIETQVEWISDTVAYAERNEI
RAIEPTPEAEEEWTQTCTDIANATLFTRGDSWIFGANVPGKKPSVLFYLGGLGNYRNVLAGVVADS
YRGFELKSAVPVTA.

A mutation site and amino acid mutation of a mutant 1(CHMO-Rs-Cv-V1) are: F508Y+F435N+L438A+T436S+F280V+S441V; and a mutation site and amino acid mutation of a mutant 2(CHMO-Rs-Cv-V2) are: F508Y+F435N+L438A+T436S+F280V+S441V+L510V.

An amino acid sequence of the CHMO-Rr cyclohexanone monooxygenase is SEQ ID NO.5:

MTTSIDREALRRKYAEERDKRIRPDGNDQYIRLDHVDGWSHDPYMPITPREPKLDHVTFAFIG
GGFSGLVTAARLRESGVESVRIIDKAGDFGGVWYWNRYPGAMCDTAAMVYMPLLEETGYMPTEK
YAHGPEILEHCQRIGKHYDLYDDALFHTEVTDLVWQEHDQRWRISTNRGDHFTAQFVGMGTGPLH
VAQLPGIPGIESFRGKSFHTSRWDYDYTGGDALGAPMDKLADKRVAVIGTGATAVQCVPELAKYCR
ELYVVQRTPSAVDERGNHPIDEKWFAQIATPGWQKRWLDSFTAIWDGVLTDPSELAIEHEDLVQDG
WTALGQRMRAAVGSVPIEQYSPENVQRALEEADDEQMERIRARVDEIVTDPATAAQLKAWFRQM
CKRPCFHDDYLPAFNRPNTHLVDTGGKGVERITENGVVVAGVEYEVDCIVYASGFEFLGTGYTDR
AGFDPTGRDGVKLSEHWAQGTRTLHGMHTYGFPNLFVLQLMQGAALGSNIPHNFVEAARVVAAIV
DHVLSTGTSSVETTKEAEQAWVQLLLDHGRPLGNPECTPGYYNNEGKPAELKDRLNVGYPAGSA
AFFRMMDHWLAAGSFDGLTFR

A mutation site and amino acid mutation of a mutant 1(CHMO-Rr-V1) are: P190L+Y559M+C249V+C393V+C257A+M45T; and a mutation site and amino acid mutation of a mutant 2(CHMO-Rr-V2) are: Y559M+P190L+P504V.

The phosphate buffer solution (PB) used in the following embodiments is a disodium hydrogen phosphate-sodium dihydrogen phosphate buffer solution.

### Embodiment 1

Transaminase TA-CV CLEA (cross-linked enzyme) is entrapped and immobilized by PVA membrane.

Preparation of a PVA I solution: 50 mL of 10%(w/v)PVA(200KDa) is mixed with 30 mL of 10% (w/v) acetic acid, 50%(v/v) methanol and 10%(w/v) sulphuric acid.

PVA membrane entrapment: the pH of the PVA I solution is adjusted to 6.0; and 20 mL of the solution is taken to mix with suspension liquid of a TA-CV cross-linked enzyme aggregate (CLEA) (the composition of the suspension liquid of a TA-CV cross-linked enzyme aggregate CLEA is 0.5 g of TA-CV cross-linked enzyme in 2 ml of 0.1M phosphate buffer solution (PB) with pH being 7.0, containing 2 mg of pyrrolaldehyde phosphate (PLP) per ml), and then stirring is performed for 20 min, to form a mixed system. The mixed system is poured in a 3D porous silica gel template, and holes in each template are square, of which volumes are about 0.1-0.2 cm³ and surface areas are about 2-5 cm²; and drying is performed at 37°C, to obtain a membrane-entrapped enzyme. The membrane-entrapped enzyme is soaked in a 0.1M PB(pH 7.0)+0.5M NaCl buffer solution for 3h; the membrane-entrapped enzyme is taken out from the buffer solution; and then, the membrane-entrapped enzyme is washed with 0.1M PB(pH 7.0) for 3 times, so as to obtain the PVA membrane immobilized enzyme in Embodiment 1.

### Comparative example 1

The 3D porous silica gel template in Embodiment 1 is replaced with a high temperature resistant silica gel plate, so as to form planar PVA membrane-entrapped enzyme. A bottom area of the silica gel plate is 80 cm².

In addition, the impact of parameters such as PVA with different molecular weights, a PVA concentration and a ratio of the enzyme to the PVA on the activity and stability of the PVA immobilized enzyme is investigated.

Conversion and stability test:

Model response used in translational research:

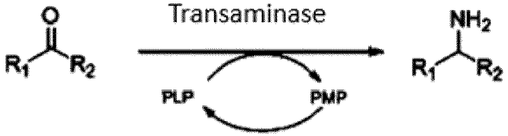

R₁ and R₂ in the above reaction formula may be independently selected from H, substituted or unsubstituted alkyl, substituted or unsubstituted cycloalkyl, substituted or unsubstituted aralkyl, substituted or unsubstituted heterocyclyl, or substituted or unsubstituted heterocycloalkyl. R₁ and R₂ may be connected into a ring.

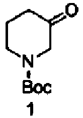

0.1 g of ketone substrate 1 is dissolved in 0.35 mL of methanol; 3.0 molar equivalents of isopropylamine hydrochloride are added as an amino donor, and 5 mg of PLP is added in a reaction system; and then, dilution is performed with 0.3 mL of 0.1M PB 7.0 so as to form a system to be reacted. 3 mg of TA-CV CLEA or the three-dimensional structured PVA membrane immobilized enzyme containing 3 mg of the TA-CV CLEA in Embodiment 1 or a planar PVA membrane immobilized enzyme containing 3 mg of the TA-CV CLEA in Comparative example 1 is used as a catalyst. After the reaction is performed at 30°C for 20 h, a conversion rate is detected by means of an HPLC method. The immobilized enzyme is separated after each reaction ends, and reused in the next reaction. The number of reuse is checked. Then, the conversion rate for 11 cycles is tested, and recorded in Table 2.

**Table 2**

| | Reuse number of conversion rate (%) | | | | | | | | | | |
|---|---|---|---|---|---|---|---|---|---|---|---|
| | 1 | 2 | 3 | 4 | 5 | 6 | 7 | 8 | 9 | 10 | 11 |
| TA-CV-CLEA | 98 | 95 | 90.7 | 98.1 | 95.3 | 93.6 | 91.9 | 90.8 | 86.6 | 80.1 | 72.4 |
| Three-dimensional structured PVA membrane immobilized enzyme | 96.2 | 93.1 | 99.3 | 96.8 | 97.9 | 97.2 | 97.3 | 96.1 | 96.3 | 95.7 | 96.1 |
| Planar PVA membrane immobilized enzyme | 84.3 | 83.1 | 83.4 | 81.9 | 81.0 | 80.8 | 80.6 | 76.9 | 76.1 | 76.0 | 75.4 |

In addition, results of the impact of the PVA molecular weight, the PVA concentration and the ratio of the enzyme to the PVA solution on the activity and stability of the PVA immobilized enzyme are shown in Table 3.

**Table 3**

| Enzyme | PVA molecular weight (KDa) | PVA concentration (g/100 mL) | Ratio of enzyme to PVA solution (g/100 mL) | Conversion rate (%) | Number of cycles |
|---|---|---|---|---|---|
| TA-CV-V1-CLEA | 6 | 15 | 5 | > 90% | 7 |
| TA-CV-V1-CLEA | 20 | 15 | 5 | > 90% | 9 |
| TA-CV-V1-CLEA | 100 | 15 | 5 | > 95% | 15 |
| TA-CV-V1-CLEA | 200 | 15 | 5 | > 95% | 15 |
| TA-CV-V1-CLEA | 250 | 15 | 5 | > 80% | 5 |
| TA-CV-V1-CLEA | 100 | 1 | 5 | > 90% | 8 |
| TA-CV-V1-CLEA | 100 | 10 | 5 | > 90% | 13 |
| TA-CV-V1-CLEA | 100 | 20 | 5 | > 90% | 17 |
| TA-CV-V1-CLEA | 20 | 50 | 5 | > 90% | 11 |
| TA-CV-V1-CLEA | 20 | 60 | 5 | > 90% | 8 |
| TA-CV-V1-CLEA | 200 | 12 | 0.5 | > 90% | 7 |
| TA-CV-V1-CLEA | 200 | 12 | 1 | > 90% | 13 |
| TA-CV-V1-CLEA | 200 | 12 | 10 | > 90% | 17 |
| TA-CV-V1-CLEA | 200 | 12 | 20 | > 90% | 16 |
| TA-CV-V1-CLEA | 200 | 12 | 40 | > 90% | 12 |
| TA-CV-V1-CLEA | 200 | 12 | 50 | > 90% | 9 |

The impact of the pH value of the mixed system on the activity and stability of the enzyme is shown in Table 4.

**Table 4**

| Enzyme | PVA molecular weight (KDa) | PVA concentration (g/100 mL) | Ratio of enzyme to PVA solution (g/100 mL) | pH | Conversion rate (%) | Number of cycles |
|---|---|---|---|---|---|---|
| TA-CV -CLEA | 200 | 15 | 5 | 4 | 50% | 2 |
| TA-CV -CLEA | 200 | 15 | 5 | 5 | 80% | 4 |
| TA-CV -CLEA | 200 | 15 | 5 | 6 | > 95% | 8 |
| TA-CV -CLEA | 200 | 15 | 5 | 6.5 | > 95% | 8 |
| TA-CV-V1-CLEA | 200 | 15 | 5 | 4 | 65% | 6 |
| TA-CV-V1-CLEA | 200 | 15 | 5 | 5 | 75% | 9 |
| TA-CV-V1-CLEA | 200 | 15 | 5 | 6 | > 95% | 17 |
| TA-CV-V1-CLEA | 200 | 15 | 5 | 6.5 | > 95% | 17 |
| TA-CV-V2-CLEA | 200 | 15 | 5 | 4 | 75% | 8 |
| TA-CV-V2-CLEA | 200 | 15 | 5 | 5 | 90% | 9 |
| TA-CV-V2-CLEA | 200 | 15 | 5 | 6 | > 95% | 18 |
| TA-CV-V2-CLEA | 200 | 15 | 5 | 6.5 | > 95% | 18 |

### Embodiment 2

Transaminase TA-CV CLEA is entrapped and immobilized by PVA membrane.

Preparation of a PVA II solution: 12%-15%(w/v)PVA aqueous solution.

PVA membrane entrapment: 30 mL of the PVA solution is taken; 3 g of CLEA wet particles and 50 mg of PLP are added, and uniform stirring is performed to form the mixed system; then the mixed system is poured to the 3D porous silica gel template, and the holes in each template are circular, of which volumes are about 0.15-0.2 cm³ and surface areas are about 3-5 cm²; and drying is performed at 37 °C , to obtain the membrane-entrapped enzyme. The membrane-entrappedenzyme is soaked in a 0.1M PB 7.0 buffer solution over night; the membrane-entrapped enzyme is taken out from the buffer solution; and then, the membrane-entrapped enzyme is washed with the 0.1M PB7.0 buffer solution for 2 times, so as to obtain the PVA membrane immobilized enzyme in Embodiment 2.

Before the CLEA is added, a certain amount of PEG 400-PEG6000 is dissolved in the PVA II solution for parallel experiment.

Conversion and stability test is performed according to a substrate type of Embodiment 1.

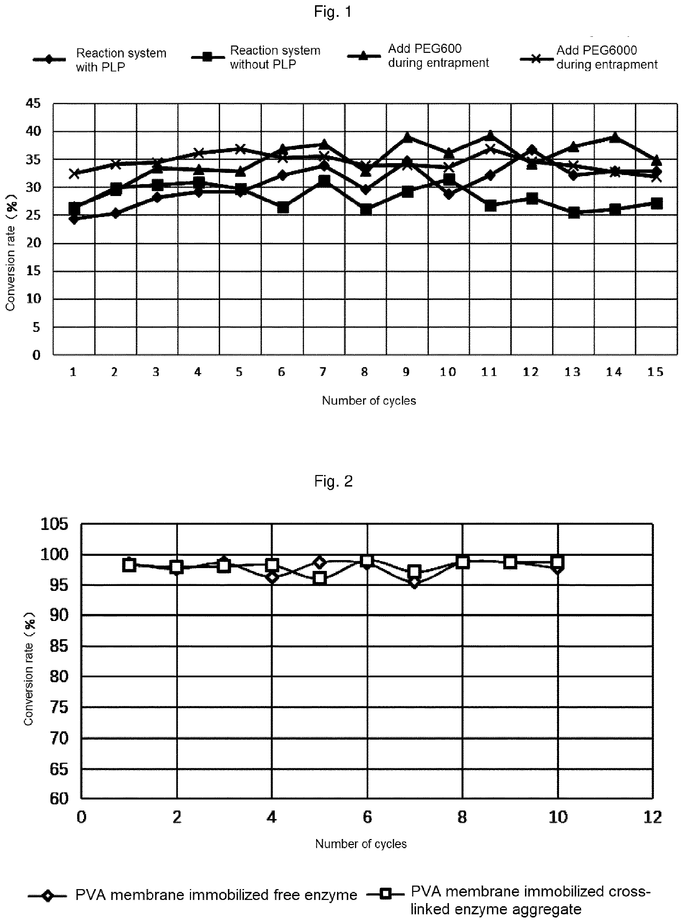

0.1 g of ketone substrate 1 is dissolved in 0.35 mL of methanol; 3.0 molar equivalents of isopropylamine hydrochloride are added as an amino donor, and 5 mg of PLP is added in a reaction system; and then, dilution is performed with 0.3 mL of 0.1M PB 7.0 so as to form a system to be reacted. Each PVA membrane immobilized enzyme in Embodiment 2 that is entrapped with 6 mg of TA-Cv CLEA and has a specific surface area of about 6 cm² is clipped and used as the catalyst, and a parallel reaction is performed without adding PLP. After the reaction is performed at 30°C for 4 h, the conversion rate is detected by means of the HPLC method. Each reaction is performed for 20 h, and the immobilized enzyme is separated after one reaction ends and reused in the next reaction. The number of reuse is checked, and test results are shown in Fig. 1.

According to Fig. 1, it may be seen that, the activity and stability of the PVA membrane immobilized enzyme in Embodiment 2 are excellent, and the activity is not reduced after the immobilized enzyme is used for 14 cycles. The activity is slightly low when the PLP is not added, but the stability is as good as the stability when the PLP is added. According to Fig. 1, it may be seen that, a reaction speed may be accelerated by adding PEG400 or PEG6000.

In addition, the impact of a PEG molecular weight and a PEG concentration in the mixed system on the activity and stability of the enzyme is investigated, and results are shown in Table 5.

**Table 5**

| Enzyme | PEG molecular weight (Da) | Concentration of added PEG | 3h conversion rate (%) | Number of cycles |
|---|---|---|---|---|
| TA-Cv-V1 cross-linked enzyme aggregate | No | No | > 25% | 14 |
| TA-Cv-V1 cross-linked enzyme aggregate | 400 | 1 | > 35% | 14 |
| TA-Cv-V1 cross-linked enzyme aggregate | 400 | 3 | > 35% | 15 |
| TA-Cv-V1 cross-linked enzyme aggregate | 400 | 5 | > 35% | 15 |
| TA-Cv-V1 cross-linked enzyme aggregate | 400 | 10 | > 35% | 14 |
| TA-Cv-V1 cross-linked enzyme aggregate | 2000 | 3 | > 35% | 15 |
| TA-Cv-V1 cross-linked enzyme aggregate | 2000 | 5 | > 40% | 15 |
| TA-Cv-V1 cross-linked enzyme aggregate | 2000 | 10 | > 40% | 15 |
| TA-Cv-V1 cross-linked enzyme aggregate | 2000 | 12 | > 40% | 13 |
| TA-Cv-V1 cross-linked enzyme aggregate | 2000 | 15 | > 40% | 10 |
| TA-Cv-V1 cross-linked enzyme aggregate | 6000 | 1 | > 35% | 14 |
| TA-Cv-V1 cross-linked enzyme aggregate | 6000 | 3 | > 35% | 14 |
| TA-Cv-V1 cross-linked enzyme aggregate | 6000 | 5 | > 35% | 14 |
| TA-Cv-V1 cross-linked enzyme aggregate | 6000 | 10 | > 35% | 14 |

### Embodiment 3

A TA-CV wet cell free enzyme or TA-CV CLEA is entrapped and immobilized by PVA-organic solvent membrane.

7.0 mL of a 10%(w/v)PVA solution is taken and mixed with 5 mL of a TA-CV free enzyme solution (which contains 5 mg/mL of the PLP) or 1 g of TA-CV CLEA; stirring is performed for 30 min to form the mixed system; the mixed system is poured into a glass or high temperature resistant plate, and is allowed to stand for 3 h; and then, 15 mL of an organic solvent acetonitrile is gently poured into the 3D porous silica gel template, and the holes in each template are circular, of which volumes are about 0.15-0.2 cm³ and surface areas are about 3-5 cm²; and drying is performed at 37°C to obtain the membrane-entrapped enzyme. The membrane-entrapped enzyme is soaked in a 0.1M PB 7.0+0.5M NaCl buffer solution for 2-3h; the membrane-entrapped enzyme is taken out from the buffer solution; and then, the membrane-entrapped enzyme is washed with 0.1M PB(pH 7.0) for 3 times, so as to obtain two PVA membrane immobilized enzymes in Embodiment 3.

Conversion and stability test is performed according to a substrate type of Embodiment 1.

0.1 g of ketone substrate 1 is dissolved in 0.35 mL of methanol; 3.0 molar equivalents of isopropylamine are added as an amino donor, and 5 mg of PLP is added in a reaction system; and then, dilution is performed with 0.3 mL of 0.1M PB 7.0 so as to form a system to be reacted. The PVA membrane immobilized enzyme that is made of entrapped TA-CV wet cells or CLEA and has a specific surface area of about 6 cm² is clipped and used as the catalyst. After the reaction is performed at 30°C for 20 h, the conversion rate is detected by means of the HPLC method. The immobilized enzyme is separated after each reaction ends and reused in the next reaction. The number of reuse is checked, and test results are shown in Table 6 and Fig. 2.

**Table 6**

| | Conversion rate | | | | | | | | | |
|---|---|---|---|---|---|---|---|---|---|---|
| Number of cycles | 1 | 2 | 3 | 4 | 5 | 6 | 7 | 8 | 9 | 10 |
| PVA membrane immobilized TA-CV free enzyme | 98.5 | 97.6 | 98.6 | 96.3 | 98.7 | 98.5 | 95.4 | 98.7 | 98.7 | 97.7 |
| CLEA-PVA membrane immobilized cross-linked enzyme aggregate (TA-CV CLEA) | 98.2 | 97.9 | 98 | 98.2 | 96 | 99 | 97.1 | 98.7 | 98.7 | 98.7 |

After 20 h of reaction, the conversion rate may still reach 98%, and is still stable after being used for 10 cycles, without losing activity.

### Embodiment 4

High specific surface area immobilized TA-CV is prepared by means of a PVA-organic solvent TA-CV entrapment.

5.0 mL of the 10%(w/v)PVA solution is taken and mixed with 5 mL of the TA-CV free enzyme solution (with enzyme concentration being 0.1 g/mL, and containing 5 mg/mL of PLP); stirring is performed for 20min to form the mixed system, and air bubbles are removed; and then 0.1 mL of the mixed system is dropped into the 3D porous silica gel template, and single holes in each template are circular or square, of which volumes are about 0.15-0.2 cm³ and surface areas are about 3-5 cm². The plate is allowed to stand for 3 h; then 0.06 mL of an organic solvent acetone or acetonitrile is gently dropped in the holes; and drying is performed at 37°C, and hollow block-shaped membrane-entrapped enzymes in the holes. The hollow block-shaped membrane-entrapped enzymes are soaked in the 0.1M PB 7.0+0.5M NaCl buffer solution for 2-3 h. Then, the buffer solution is removed, and the hollow block-shaped membrane-entrapped enzymes are washed with 0.1M PB7.0 for 3 times, so as to obtain the PVA membrane immobilized enzymes in Embodiment 4.

Partial membrane-entrapped enzyme is taken and further modified by glutaraldehyde. The membrane-entrapped enzyme is resuspended with 0.1M PB 7.0, the glutaraldehyde is added dropwise, and 1-2 g of glutaraldehyde is added dropwise to every 100 mL of suspension liquid; stirring is performed for 2 h at room temperature; the buffer solution is removed; and the hollow block-shaped membrane-entrapped enzyme modified by the glutaraldehyde is washed with 0.1M PB 7.0 for 3 times, so as to obtain the glutaraldehyde-modified PVA membrane immobilized enzyme.

Reaction activity and stability test is performed according to the substrate type of Embodiment 1.

0.1 g of ketone substrate is dissolved in 0.35 mL of methanol; 3.0 molar equivalents of isopropylamine hydrochloride are added as an amino donor, and 5 mg of the cofactor PLP is added in the reaction system; and then, dilution is performed with 0.3 mL of 0.1M PB 7.0 so as to form the system to be reacted. The PVA membrane immobilized enzyme that is entrapped with the TA-CV free enzyme (dried with acetone) and has a specific surface area of about 6 cm², the PVA membrane immobilized enzyme that is entrapped with the TA-CV free enzyme (dried with the acetone and modified by glutaraldehyde) and has a specific surface area of about 6 cm², the PVA membrane immobilized enzyme that is entrapped with the TA-CV free enzyme (dried with acetonitrile) and has a specific surface area of about 6 cm², and the PVA membrane immobilized enzyme that is entrapped with the TA-CV free enzyme (dried with acetonitrile and modified by glutaraldehyde) and has a specific surface area of about 6 cm² are clipped and used as catalysts.

After the reaction is performed at 30°C for 20 h and repeated for 10 cycles, the conversion rate is detected by means of the HPLC method after 20 h of reaction. The immobilized enzyme is separated after each reaction ends and reused in the next reaction. The number of reuse is checked, and test results are shown in Table 7.

**Table 7**

| Catalyst | Drying and modification mode | Conversion rate (%) | | | | | | | | | |
|---|---|---|---|---|---|---|---|---|---|---|---|
| | | 1 | 2 | 3 | 4 | 5 | 6 | 7 | 8 | 9 | 10 |
| TA-CV | Dried with acetonitrile | 95.1 | 95.9 | 90.5 | 82.1 | 81.6 | 86.5 | 86.5 | 92.5 | 90.6 | 91.3 |
| TA-CV | Dried with acetonitrile and modified by 1% glutaraldehyde | 78.6 | 89.7 | 86.2 | 70.7 | 89.9 | 79.8 | 82.3 | 88 | 87.7 | 85.4 |
| TA-CV | Dried with acetonitrile and modified by 2% glutaraldehyde | 79.7 | 88.9 | 87.3 | 80.1 | 89.5 | 83.2 | 85.6 | 88.5 | 88.1 | 87.2 |
| TA-CV | Dried with acetonitrile and modified by 2.5% glutaraldehyde | 74.1 | 66.5 | 60.4 | 43.2 | -/- | -/- | -/- | -/- | -/- | -/- |
| TA-CV | Dried with acetone | 95.8 | 91.7 | 88.8 | 87.2 | 92.4 | 84.2 | 83.4 | 90.8 | 82.9 | 88.6 |
| TA-CV | Dried with acetone and modified by 2% glutaraldehyde | 85.3 | 78.9 | 77.9 | 72.7 | 77 | 74.2 | 78.5 | 81.2 | 81.7 | 79.3 |

In addition, the impact of a ratio of a dehydrating agent to the PVA-enzyme mixed system on the activity and stability of the enzyme is investigated, and results are shown in Table 8.

**Table 8**

| Catalyst | Dehydrating agent | Volume ratio of dehydrating agent to PVA-enzyme mixed system | Conversion rate (%) | Reuse number/time |
|---|---|---|---|---|
| TA-CV | Acetonitrile | 1: 20 | > 85% | 16 |
| | | 1: 10 | > 85% | 18 |
| | | 1: 5 | > 85% | 19 |
| | | 1: 1 | > 85% | 19 |
| | | 3: 1 | > 85% | 19 |
| | | 5: 1 | > 85% | 18 |
| | | 7: 1 | > 85% | 16 |
| | Acetone | 1: 20 | > 85% | 15 |
| | | 1: 10 | > 85% | 17 |
| | | 1: 5 | > 85% | 19 |
| | | 1: 1 | > 85% | 19 |
| | Ethanol | 1: 10 | > 85% | 18 |
| | | 1: 5 | > 85% | 19 |
| | | 1: 1 | > 85% | 19 |

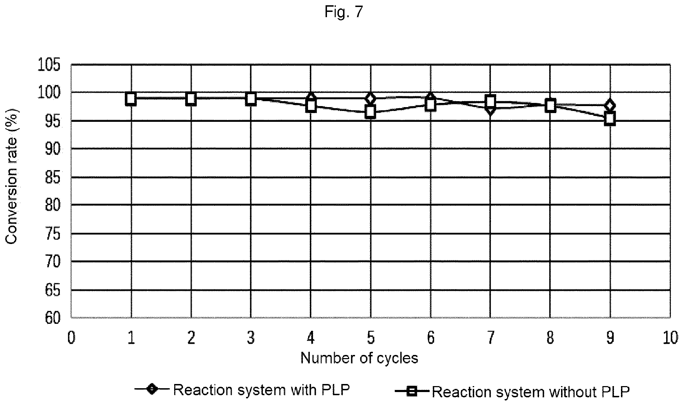

According to data in Fig. 7, it may be seen that, when the PVA membrane immobilized enzyme in Embodiment 4 is used as the catalyst, the activity is not reduced after 10 cycles. When being modified by GA, although the activity of the enzyme is slightly low, the stability is as good as the stability without being modified by GA.

### Embodiment 5

A TA-CV free enzyme is entrapped and immobilizedby PVA-CFP membrane.

PVA solution: a 12%(w/v) solution is prepared in water.

Cofactor-polymer solution (CFP solution): 2%w/v polyethyleneimine (PEI)(3KDa ~ 70KDa) is dissolved, 5 mg/mL of the cofactor (PLP) is added, and mixing is performed for 0.5-3 h at room temperature.

35 mL of the PVA solution and 5 mL of a CFP solution are taken to well mix for 30 min; then 5 mL of the enzyme solution (the enzyme concentration being 0.1 g/mL) and 3-5 mg of the cofactor PLP are added; and mixing is performed for 30 min at room temperature, so as to form the mixed system. Then, the mixed system is poured to the 3D structured porous silica gel template, and dried at 37°C to obtain the membrane-entrapped enzyme. The single holes in each template are circular or square, of which volumes are about 0.15-0.2 cm³ and surface areas are about 3-5 cm². The membrane-entrapped enzyme is soaked in the 0.1M PB 7.0 buffer solution over night. After the buffer solution is removed, the membrane-entrapped enzyme is washed with 0.1M PB7.0 for 2 times, so as to obtain the PVA membrane immobilized enzyme in Embodiment 5.

Activity and stability test is performed according to the substrate type of Embodiment 1.

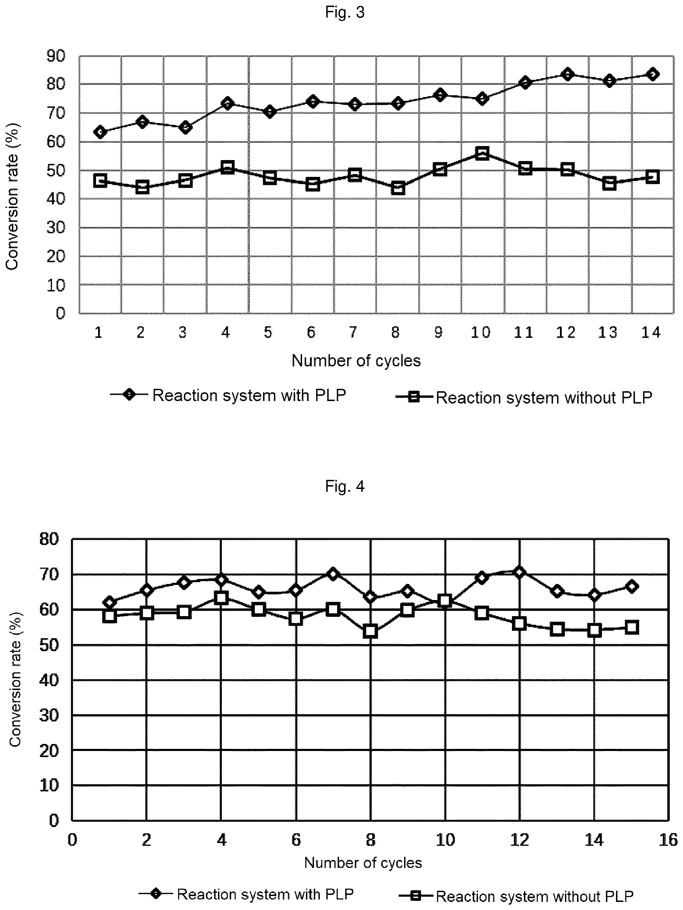

0.1 g of ketone substrate 1 is dissolved in 0.35 mL of methanol; 3.0 molar equivalents of isopropylamine are added as an amino donor, and 5 mg of PLP is added in a reaction system; and then, dilution is performed with 0.3 mL of 0.1M PB 7.0 so as to form a system to be reacted. The PVA membrane immobilized enzyme in Embodiment 4 that is entrapped with 3mg of TA-CV free enzyme and has a specific surface area of about 3-4 cm² is clipped and used as the catalyst, and a parallel reaction is performed without adding PLP. After the reaction is performed at 30°C for 20 h and repeated for 14 cycles, the conversion rate is detected by means of the HPLC method after 4 h of reaction, and test results are shown in Fig. 3.

According to Fig. 3, it may be seen that, the activity and stability of the formed PVA membrane immobilized enzyme are excellent, and the activity is not reduced after 14 cycles. The activity is slightly low when the PLP is not added, but the stability is as good as the stability when the PLP is added.

In addition, the impact of a PEI molecular weight and a PEI concentration in the mixed system on the activity and stability of the enzyme is investigated, and results are shown in Table 9.

**Table 9**

| Enzyme | PEI molecular weight (KDa) | Concentration of added PEI | 4h conversion rate (%) | Number of cycles |
|---|---|---|---|---|
| TA-Cv-V2 | No | No | > 25% | 14 |
| TA-Cv-V2 | 0.6 | 0.3 | > 25% | 14 |
| TA-Cv-V2 | 3 | 0.3 | > 35% | 16 |
| TA-Cv-V2 | 10 | 0.3 | > 35% | 18 |
| TA-Cv-V2 | 25 | 0.3 | > 35% | 16 |
| TA-Cv-V2 | 50 | 0.3 | > 35% | 75 |
| TA-Cv-V2 | 60 | 0.3 | > 35% | 18 |
| TA-Cv-V2 | 70 | 0.3 | > 35% | 15 |
| TA-Cv-V2 | 100 | 0.3 | > 35% | 11 |
| TA-Cv-V2 | 3 | 0.05 | > 35% | 15 |
| TA-Cv-V2 | 3 | 0.1 | > 35% | 17 |
| TA-Cv-V2 | 3 | 0.5 | > 35% | 16 |
| TA-Cv-V2 | 3 | 0.8 | > 35% | 16 |
| TA-Cv-V2 | 3 | 1 | > 35% | 15 |
| TA-Cv-V2 | 60 | 0.05 | > 35% | 15 |
| TA-Cv-V2 | 60 | 0.3 | > 35% | 18 |
| TA-Cv-V2 | 60 | 0.5 | > 35% | 18 |
| TA-Cv-V2 | 60 | 1 | > 35% | 14 |
| TA-Cv-V2 | 60 | 2 | > 35% | 10 |

### Embodiment 6

TA-CV CLEA is entrapped and immobilized by PVA-CFP membrane.

PVA solution: a 12%w/v solution is prepared in water.

CFP solution: a 2%w/v PEI solution (3KDa-70KDa) is prepared in the water, 5 mg/mL of the cofactor (PLP) is added, and mixing is performed for 0.5-3 h at room temperature.

3 g of TA-CV CLEA is suspended in 10 mL of the CFP solution, and is well mixed with 30 mL of the PVA solution, so as to form the mixed system. Then, the mixed system is poured to the porous silica gel template, and dried at 37°C to form the membrane-entrapped enzyme. The single holes in each template are circular or square, of which volumes are about 0.15-0.2 cm³ and surface areas are about 3-5 cm². The membrane-entrapped enzyme is soaked in the 0.1M PB 7.0 buffer solution over night. The membrane-entrapped enzyme is taken out from the buffer solution and then washed with 0.1M PB7.0 for 2 times, so as to obtain the PVA membrane-entrapped immobilized enzyme in Embodiment 6.

Activity and stability test is performed according to the substrate type of Embodiment 1.

0.1 g of ketone substrate 1 is dissolved in 0.35 mL of methanol; 3.0 molar equivalents of isopropylamine are added as an amino donor, and 5 mg of PLP is added in a reaction system; and then, dilution is performed with 0.3 mL of 0.1M PB 7.0 so as to form a system to be reacted. The PVA membrane-entrapped immobilized enzyme in Embodiment 6 that is entrapped with 6 mg of TA-CV CLEA and has a specific surface area of about 5cm² is used as the catalyst, and a parallel reaction is performed without adding PLP. After the reaction is performed at 30°C for 20 h and repeated for 14 cycles, the conversion rate is detected by means of the HPLC method. The immobilized enzyme is separated after each reaction ends and reused in the next reaction. The number of reuse is checked, and test results are shown in Fig. 4.

According to Fig. 4, it may be seen that, the activity and stability of the PVA membrane-entrapped immobilized enzyme are excellent, and the activity is not reduced after the immobilized enzyme is used for 14 cycles. The activity is slightly low when the PLP is not added, but the stability is as good as the stability when the PLP is added.

### Embodiment 7

A TA-Ac free enzyme is entrapped and immobilized by PVA-CFP membrane.

PVA solution: a 12%w/v solution is prepared in water.

CFP solution: a 2%w/v PEI solution (3KDa-70KDa) is prepared in the water, 5 mg/mL of the cofactor (PLP) is added, and mixing is performed for 0.5-3 h at room temperature.

35 mL of the PVA solution and 5 mL of the CFP solution are taken to well mix for 30 min; then 5 mL of the TA-Ac enzyme solution (the enzyme concentration being 0.1) and 3-5 mg of the cofactor PLP are added; and mixing is performed for 30 min at room temperature, so as to obtain the mixed system. Then, the mixed system is poured in the 3D porous silica gel template, and the single holes in each template are circular or square, of which volumes are about 0.15-0.2 cm³ and surface areas are about 3-5 cm²; and drying is performed at 37°C, to obtain the membrane-entrapped enzyme.

The membrane-entrapped enzyme is soaked in the 0.1M PB 7.0 over night. The membrane-entrapped enzyme is taken out from the buffer solution and then washed with 0.1M PB7.0 for 2 times, so as to obtain the PVA membrane-entrapped immobilized enzyme in Embodiment 7.

Activity and stability test is performed according to the substrate type of Embodiment 1.

Specifically, a ketone substrate 2 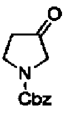 is used to replace the ketone substrate 1 used in Embodiment 1.

A test is performed in an aqueous buffer system. 0.1 g of the ketone substrate 2 is taken and suspended in 1 mL of the 0.1M PB 7.0 buffer solution; 3.0 molar equivalents of isopropylamine hydrochloride are added as an amino donor, and 5 mg of the PLP is added in the reaction system; and the PVA membrane-entrapped immobilized enzyme in Embodiment 7 that contains 10 mg of the TA-Ac free enzyme is used as the catalyst. After the reaction is performed at 30°C for 20 h, 5 cycles are repeated, and the conversion rate is detected by means of the HPLC method. The immobilized enzyme is separated after each reaction ends and reused in the next reaction. The number of reuse is checked, and test results are shown in Table 10.

A test is performed in a biphasic system. 0.1 g of the ketone substrate 2 is taken and dissolved in 1 mL of MTBE as a non-aqueous phase; 1 mL of 0.1M PB 7.0 is added as an aqueous phase; 3.0 molar equivalents of isopropylamine are added as the amino donor, and 5 mg of the PLP is added in the reaction system; and the PVA membrane immobilized enzyme in Embodiment 7 that contains 10 mg of the TA-Ac free enzyme is used as the catalyst. After the reaction is performed at 30°C for 20 h, 5 cycles are repeated, and the conversion rate is detected by means of the HPLC method. The immobilized enzyme is separated after each reaction ends and reused in the next reaction. The number of reuse is checked, and test results are shown in Table 10.

**Table 10**

| | Solvent system | Conversion rate (%) | | | | |
|---|---|---|---|---|---|---|
| | | 1 | 2 | 3 | 4 | 5 |
| PVA membrane immobilized enzyme TA-Ac in Embodiment 7 | Aqueous | 63.7 | 67.4 | 59.5 | 64.1 | 62.9 |
| | Biphasic | 39.6 | 42.2 | 46.4 | 49.2 | 44.1 |

According to data in Table 10, it may be seen that, the activity and stability of the PVA membrane immobilized enzyme in Embodiment 7 are excellent, and the activity is not reduced after the immobilized enzyme is used for 5 cycles.

The impact of the PVA molecular weight, the PVA concentration and the ratio of the enzyme to the PVA solution on the activity and stability of the enzyme is investigated, and results are shown in Table 11.

**Table 11**

| Enzyme | PVA molecular weight (KDa) | PVA concentration (g/100 mL) | Ratio of enzyme to PVA solution (g/100 mL) | Conversion rate (%) | Number of cycles |
|---|---|---|---|---|---|
| TA-Ac-V1 | 20 | 15 | 5 | > 60% | 8 |
| TA-Ac-V1 | 100 | 15 | 5 | > 60% | 13 |
| TA-Ac-V1 | 200 | 15 | 5 | > 60% | 14 |
| TA-Ac-V1 | 250 | 15 | 5 | > 60% | 7 |
| TA-Ac-V1 | 100 | 1 | 5 | > 60% | 8 |
| TA-Ac-V1 | 100 | 10 | 5 | > 60% | 10 |
| TA-Ac-V1 | 100 | 20 | 5 | > 60% | 11 |
| TA-Ac-V1 | 20 | 50 | 5 | > 60% | 13 |
| TA-Ac-V1 | 20 | 60 | 5 | > 60% | 8 |
| TA-Ac-V1 | 200 | 12 | 0.5 | > 60% | 7 |
| TA-Ac-V1 | 200 | 12 | 1 | > 60% | 9 |
| TA-Ac-V1 | 200 | 12 | 10 | > 60% | 12 |
| TA-Ac-V1 | 200 | 12 | 20 | > 60% | 13 |
| TA-Ac-V1 | 200 | 12 | 40 | > 60% | 11 |
| TA-Ac-V1 | 200 | 12 | 50 | > 60% | 7 |
| TA-Ac | 200 | 12 | 10 | > 60% | 5 |
| TA-Ac | 200 | 12 | 20 | > 60% | 6 |
| TA-Ac | 200 | 12 | 40 | > 60% | 6 |

### Embodiment 8

Ketoreductase is entrapped and immobilized by PVA-CFP membrane.

PVA solution: a 12%(w/v) solution is prepared in water.

CFP solution: a 2%w/v PEI (3KDa-70KDa) dissolved in the water is prepared, 5 mg/mL of the cofactor (NAD⁺ or PLP) is added as appropriate, and mixing is performed for 0.5-3 h at room temperature.

35 mL of the PVA solution and 5 mL of the CFP solution are taken to well mix for 30 min; then 5 mL of a KRED-Ac or KRED-Cm enzyme solution (the enzyme concentration being 0.1 g/mL) and 4 mg of the cofactor NAD⁺ are added; and mixing is performed for 30 min at room temperature, so as to form the mixed system. Then, the mixed system is poured to a 3D siloxane template, and the single holes in each template are circular or square, of which volumes are about 0.15-0.2 cm³ and surface areas are about 3-5 cm². Drying is performed at 37°C, to form the membrane-entrapped enzyme. The membrane-entrapped enzyme is soaked in the 0.1M PB 7.0 over night. The membrane-entrapped enzyme is taken out from the buffer solution and then washed with 0.1M PB7.0 for 2 times, so as to obtain the PVA membrane immobilized enzyme containing NAD⁺ in Embodiment 8.

The PLP is used to replace the cofactor NAD⁺, and the above process is repeated, so as to obtain the PVA membrane immobilized enzyme containing PLP in Embodiment 8.

### Activity and stability test:

Model response used in translational research:

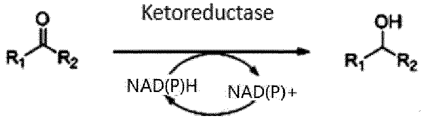

R1 and R2 in the above reaction formula may be independently selected from H, substituted or unsubstituted alkyl, substituted or unsubstituted cycloalkyl, substituted or unsubstituted aralkyl, substituted or unsubstituted heterocyclyl, or substituted or unsubstituted heterocycloalkyl. R1 and R2 may be connected into a ring.

Ketone substrate:

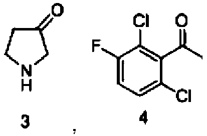

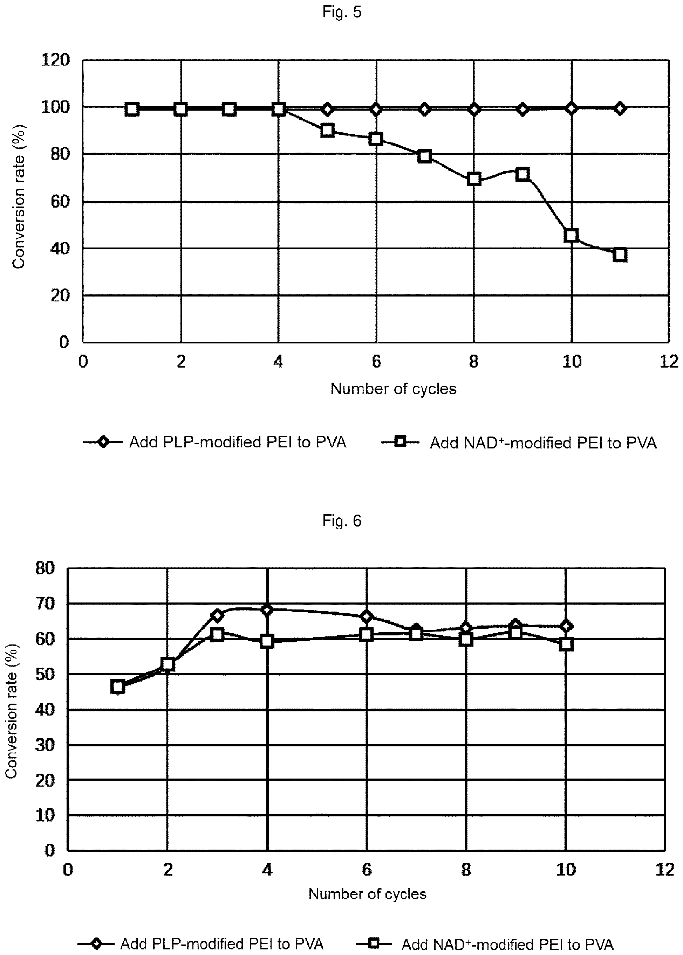

0.1 g of a ketone substrate 3 or 4 is taken and dissolved in 0.5 mL of isopropyl alcohol; 0.5 mL of 0.1M PB 7.0 containing 5 mg of the cofactor NAD⁺ is added in the reaction system, so as to form the system to be reacted; and the PVA membrane-entrapped immobilized enzyme in Embodiment 8 that entrapped with 30 mg of ketoreductase KRED-Ac (of which activity is tested by ketone substrate 3) or ketoreductase KRED-Cm (of which activity is tested by ketone substrate 4) is applied to the system to be reacted and used as the catalyst. After the reaction is performed at 30°C for 20 h and repeated for 11 cycles, the conversion rate is detected by means of a GC method. The immobilized enzyme is separated after each reaction ends and reused in the next reaction. The number of reuse is checked, and test results are shown in Fig. 5 and Fig. 6. A substrate corresponding to Fig. 5 is the ketone substrate 3, and a substrate corresponding to Fig. 6 is the ketone substrate 4.

According to Fig. 5, it may be seen that, the activity and stability of the PVA membrane immobilized enzyme KRED-Ac in Embodiment 8 against the ketone substrate 3 are excellent, the conversion rate reaches 99%, and there is no activity loss after the immobilized enzyme is used for 11 cycles. When the CFP solution is prepared with PLP, the activity is much better than that of NAD⁺.

According to Fig. 6, it may be seen that, the activity and stability of the PVA membrane-entrapped immobilized enzyme KRED-Cm in Embodiment 8 are excellent, the conversion rate reaches 99%, and there is no activity loss after the immobilized enzyme is used for 10 cycles. When the CFP solution is prepared with PLP, the activity is much better than that of NAD⁺.

The concentration of the cofactor in the enzyme system is investigated, and results are shown in Table 12.

**Table 12**

| Enzyme | Cofactor | Cofactor concentration (mg/mL) | Conversion rate (%) | Number of cycles |
|---|---|---|---|---|
| KRFD-Ac | PLP | 0.5 | > 99% | 12 |
| KRFD-Ac | PLP | 1 | > 99% | 14 |
| KRED-Ac | PLP | 5 | > 99% | 14 |
| KRED-Ac | PLP | 10 | > 99% | 14 |
| KRED-Ac | PLP | 20 | > 99% | 14 |
| KRFD-Ac | PLP | 30 | > 99% | 14 |
| KRED-Ac | NAD | 0.5 | > 99% | 8 |
| KRED-Ac | NAD | 1 | > 99% | 10 |
| KRED-Ac | NAD | 5 | > 99% | 14 |
| KRED-Ac | NAD | 10 | > 99% | 15 |
| KRED-Ac | NAD | 20 | > 99% | 15 |
| KRED-Ac | NAD | 30 | > 99% | 15 |
| KFRD-Ac-V1 | PLP | 5 | > 99% | 18 |
| KERD-Ac-V1 | PLP | 10 | > 99% | 18 |
| KERD-Ac-V1 | NAD | 5 | > 99% | 15 |
| KERD-Ac-V1 | NAD | 10 | > 99% | 17 |

### Embodiment 9

A co-crosslinking enzyme of transaminase TA-Bt and coenzymes LDH and FDH thereof are entrapped and immobilized by PVA membrane, and the co-crosslinking enzyme of transaminase TA-Bt and coenzymes LDH and FDH thereof are hereinafter referred to as co-crosslinking enzyme.

The preparation of a PVAI solution: 50 mL of 10%(w/v)PVA(200KDa) is mixed with 30 mL of 10% (w/v) acetic acid, 50%(v/v) methanol and 10%(w/v) sulphuric acid.

PVA membrane entrapment: the pH of the PVA I solution is adjusted to 4-6.5; and 20 mL of the solution is taken to mix with suspension liquid of the co-crosslinking enzyme (the composition of the suspension liquid of the co-crosslinking enzyme is 0.5 g of the co-crosslinking enzyme of TA-Bt, LDH and FDH in 2 ml of 0.1M phosphate buffer solution (PB) with pH being 7.0, containing 2 mg of PLP per ml), and then stirring is performed for 20 min, to form the mixed system. The mixed system is poured in a 3D porous silica gel template, and holes in each template are square, of which volumes are about 0.1-0.2 cm³ and surface areas are about 2-5 cm²; and drying is performed at 37°C, to obtain a membrane-entrapped enzyme. The membrane-entrapped enzyme is soaked in a 0.1M PB(pH 7.0)+0.5M NaCl buffer solution for 3h; the membrane-entrapped enzyme is taken out from the buffer solution; and then, the membrane-entrapped enzyme is washed with 0.1M PB(pH 7.0) for 3 times, so as to obtain the PVA membrane immobilized enzyme in Embodiment 1.

Activity and stability are tested.

A reaction model used in translational research:

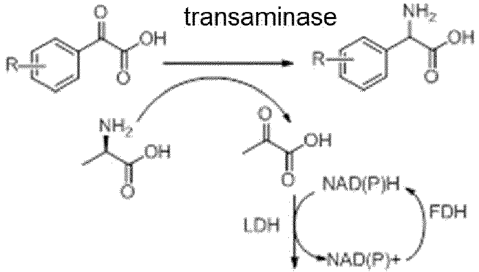

R in the above reaction formula may be selected from H, substituted or unsubstituted alkyl, substituted or unsubstituted cycloalkyl, substituted or unsubstituted aralkyl, substituted or unsubstituted heterocyclyl, substituted or unsubstituted heterocycloalkyl, or halogen.

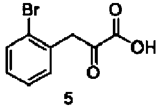

5 mL of 0.1M PB (pH 8.0) is put in a 10 ml reaction flask, 100 mg of the substrate 5, 80 mg of ammonium formate and 5 mg of PLP are subsequently added, and the pH is adjusted to pH 7.5-8.0; and then, 5 mg of NAD⁺ and 10 mg of the membrane immobilized enzyme (wet, containing 50-80% water) are added. After the reaction is performed at 30°C for 20 h, the conversion rate is detected.

The immobilized enzyme is separated after each reaction ends and reused in the next reaction. The number of reuse is checked. Specifically, the impact of a ratio of main enzyme to coenzyme in the TA-Bt and coenzyme co-immobilized enzyme on the activity and stability of the enzyme is investigated, and results are shown in Table 13.

**Table 13**

| Enzyme | Mass ratio of TA:LDH:FDH | Conversion rate (%) | Number of cycles |
|---|---|---|---|
| TA-Bt+LDH+FDH | 15:1:1 | 80% | 10 |
| TA-Bt+LDH+FDH | 10:1:1 | > 99% | 10 |
| TA-Bt+LDH+FDH | 10:1:2 | > 99% | 11 |
| TA-Bt+LDH+FDH | 5:1:1 | > 99% | 16 |
| TA-Bt+LDH+FDH | 7:1:1 | > 99% | 12 |
| TA-Bt+LDH+FDH | 7:1:2 | > 99% | 15 |
| TA-Bt+LDH+FDH | 5:1:1 | > 99% | 12 |
| TA-Bt+LDH+FDH | 6:1:2 | > 99% | 15 |
| TA-Bt+LDH+FDH | 5:1:3 | > 99% | 16 |

### Embodiment 10

TA-Bt and coenzymes D-LDH and FDH are co-immobilized by means of PVA-CFP membrane entrapment.

PVA (200KDa) solution: a 12%(w/v) solution is prepared in water.

CFP solution: a 2%(w/v)PEI(3KDa~70KDa) aqueous solution is prepared, 5 mg/mL of the cofactor (PLP) is added, and mixing is performed for 0.5-3 h at room temperature.

35 mL of the PVA solution and 5 mL of the CFP solution are taken to well mix for 30 min; then 5 mL of a TA-Bt enzyme solution (the enzyme concentration being 0.08g/mL), 0.08 g of the coenzyme D-LDH, 0.1 g of the coenzyme FDH, and 4 mg of the cofactor NAD⁺ are added; and mixing is performed for 30 min at room temperature, so as to form the mixed system. Then, the mixed system is poured to a 3D siloxane template, and the single holes in each template are circular or square, of which volumes are about 0.15-0.2 cm³ and surface areas are about 3-5 cm². Drying is performed at 37°C, to form the membrane-entrapped enzyme. The membrane-entrapped enzyme is soaked in the 0.1M PB 7.0 over night. The membrane-entrapped enzyme is taken out from the buffer solution and then washed with 0.1M PB7.0 for 2 times, so as to obtain the PVA-CFP membrane immobilized enzyme containing PLP and NAD⁺ in Embodiment 10.

Activity and stability test is performed by using a model in Embodiment 9.

5 mLof 0.1M PB (pH 8.0) is put in the 10 ml reaction flask, 100 mg of the substrate 5, 80 mg of ammonium formate and 5 mg of PLP are subsequently added, and the pH is adjusted to pH 7.5-8.0; and then, 5 mg of NAD⁺ and 10 mg of the PVA-CFP membrane immobilized enzyme containing PLP and NAD⁺ (wet, containing 50-80% water) are added. After the reaction is performed at 30°C for 20 h, the conversion rate is detected. The immobilized enzyme is separated after each reaction ends and reused in the next reaction. The number of reuse is checked, and the parallel reaction is performed without adding PLP.

After the reaction is performed at 30°C for 4 h, 9 cycles are repeated, and the conversion rate is detected by means of the HPLC method. The immobilized enzyme is separated after each reaction ends and reused in the next reaction. The number of reuse is checked, and test results are shown in Fig. 7.

According to Fig. 7, it may be seen that, the activity and stability of the obtained PVA membrane immobilized enzyme are excellent, there is no activity loss after the immobilized enzyme is used for 9 cycles, and the activity and stability of the reaction system are as good as the activity and the stability with and without PLP.

### Embodiment 11

Cyclohexanone monooxygenase is immobilized by PVA-CFP membrane.

The immobilization method is the same as that in Embodiment 10, except that the PVA-encapsulated enzyme is changed to cyclohexanone monooxygenase CHMO-Rs or CHMO-Bp, or may be a mixed enzyme of the cyclohexanone monooxygenase CHMO thereof and coenzyme alcohol dehydrogenase ADH -Tb or glucose dehydrogenase GDH, and the specific enzyme composition is shown in Table 14.

Activity and stability test:

A reaction model used in translational research:

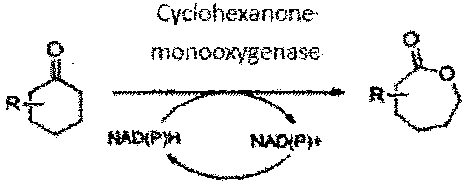

R in the above reaction formula may be selected from H, substituted or unsubstituted alkyl, substituted or unsubstituted cycloalkyl, substituted or unsubstituted aryl, substituted or unsubstituted aralkyl, substituted or unsubstituted heterocyclyl, or substituted or unsubstituted heterocycloalkyl. Alternatively, a fused aromatic ring is formed by R and a heterocyclic ring connected to R.

The activity of the PVA-CFP membrane immobilized enzyme of CHMO is detected by following substrate 6 to perform reaction.

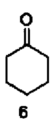

3 mL of 0.1M PB(pH 8.0) is put into the 10 ml reaction flask, 50 mg of the substrate 6, 100 mg of glucose, 5 mg of NADP⁺, 50 mg of alcohol dehydrogenase ADH-Tb and 5 mg of glucose dehydrogenase GDH are subsequently added; and then, the PVA-CFP membrane immobilized enzyme containing 20 mg of cyclohexanone monooxygenase is added. After the reaction is performed at 30°C for 20 h, the conversion rate is detected. The immobilized enzyme is separated after each reaction ends and reused in the next reaction. The number of reuse is checked.

The activity of the PVA-CFP membrane immobilized enzyme of CHMO and coenzyme GDH is detected by means of the following reaction conditions.

3 mL of 0.1M PB(pH 8.0) is put into the 10 ml reaction flask, 50 mg of the substrate 6, 100 mg of glucose and 5 mg of NADP⁺ are subsequently added; and then, 30mg of co-immobilized enzyme of CHMO and GDH mixed enzyme is added. After the reaction is performed at 30°C for 20 h, the conversion rate is detected. The immobilized enzyme is separated after each reaction ends and reused in the next reaction. The number of reuse is checked.

The activity of the PVA-CFP membrane immobilized enzyme of CHMO and coenzyme ADH-Tb is detected by means of the following reaction conditions.

3 mL of 0.1M PB(pH 8.0) is put into the 10 ml reaction flask, 50 mg of the substrate 6, 200 µl of isopropyl alcohol and 5 mg of NADP⁺ are subsequently added; and then, 30mg of co-immobilized enzyme of CHMO and ADH mixed enzyme is added. After the reaction is performed at 30°C for 20 h, the conversion rate is detected. The immobilized enzyme is separated after each reaction ends and reused in the next reaction. The number of reuse is checked.

Test results are shown in Table 14.

**Table 14**

| CFP-PVA-encapsulated enzyme | Main enzyme:coenzyme (weight ratio) | Conversion rate (%) | Number of cycles |
|---|---|---|---|
| CHMO-Rs | -/- | > 90% | 8 |
| CHMO-Bp | -/- | > 90% | 7 |
| CHMO-Rs+ADH | 12:1 | > 90% | 6 |
| | 10:1 | > 90% | 7 |
| | 5:1 | > 90% | 8 |
| | 5:2 | > 90% | 8 |
| | 1:1 | > 90% | 5 |
| | 1:2 | 80% | 3 |
| CHMO-Rs+GDH | 5:1 | > 90% | 6 |
| | 5:2 | > 90% | 7 |
| | 1:1 | > 90% | 3 |
| CHMO-Rs-V1+ADH | 5:2 | > 90% | 16 |
| CHMO-Rs-V2+ADH | 5:2 | > 90% | 18 |
| CHMO-Bp+ADH | 10:1 | > 90% | 8 |
| | 5:1 | > 90% | 10 |
| | 5:2 | > 90% | 10 |
| | 1:1 | > 90% | 5 |
| CHMO-Bp+GDH | 5:1 | > 90% | 8 |
| | 5:2 | > 90% | 7 |
| | 1:1 | > 90% | 5 |
| CHMO-Rr | -/- | > 90% | 11 |
| CHMO-Rr-V1 | -/- | > 90% | 16 |
| CHMO-Rr-V2 | -/- | > 90% | 19 |
| CHMO-Rr-V1+ADH | 10:1 | > 90% | 13 |
| CHMO-Rr-V1+ADH | 5:1 | > 90% | 16 |
| CHMO-Rr-V1+ADH | 3:1 | > 90% | 15 |
| CHMO-Rr-V2+ADH | 5:1 | > 90% | 20 |

### Embodiment 12

Alkene reductase is immobilized by PVA-CFP membrane.

The immobilization method is the same as that in Embodiment 10, except that the PVA-encapsulated enzyme is changed to alkene reductase ERED-Sc or ERED-Chr, or may be a mixed enzyme of the alkene reductase and coenzyme glucose dehydrogenase GDH thereof or ammonium formate dehydrogenase FDH, and a weight ratio of the two enzymes is ERED:GDH (or FDH)=5:1.

Activity and stability are tested.

A reaction model used in translational research:

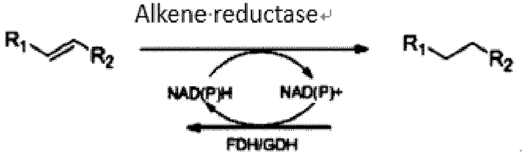

R1 and R2 in the above reaction formula may be independently selected from H, substituted or unsubstituted alkyl, substituted or unsubstituted cycloalkyl, substituted or unsubstituted aralkyl, substituted or unsubstituted heterocyclyl, or substituted or unsubstituted heterocycloalkyl. Alternatively, R1 and R2 are connected into a ring.

The activity of the PVA-CFP membrane immobilized enzyme of ERED is detected by means of reaction of the following substrate 7.

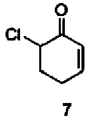

3 mL of 0.1M PB(pH7.0-8.0) is put into the 10 ml reaction flask, 100 mg of the substrate 7 is subsequently added, and then 20 mg of NAD(P)⁺, 80 mg of ammonium formate, 5 mg of FDH, and the PVA-CFP membrane immobilized enzyme containing 30 mg of alkene reductase are added. After the reaction is performed at 30°C for 16 h, the conversion rate is detected. The immobilized enzyme is separated after each reaction ends and reused in the next reaction. The number of reuse is checked.

The activity of the PVA-CFP membrane immobilized enzyme of ERED and FDH is detected by means of reaction of the following substrate 7.

3 mL of 0.1M PB(pH7.0-8.0) is put into the 10 ml reaction flask, 100 mg of the substrate 7 is subsequently added, and then 20 mg of NAD(P)⁺, 80 mg of ammonium formate, and the PVA-CFP membrane immobilized enzyme containing 40 mg of the mixed enzyme of alkene reductase and FDH are added. After the reaction is performed at 30°C for 16 h, the conversion rate is detected. The immobilized enzyme is separated after each reaction ends and reused in the next reaction. The number of reuse is checked.

The activity of the PVA-CFP membrane immobilized enzyme of ERED and GDH is detected by means of the following reaction.

3 mL of 0.1M PB(pH7.0-8.0) is put into the 10 ml reaction flask, 100 mg of the substrate 7 is subsequently added, and then 20 mg of NAD(P)⁺, 120 mg of glucose, and the PVA-CFP membrane immobilized enzyme containing 40 mg of the mixed enzyme of alkene reductase and GDH are added. After the reaction is performed at 30°C for 16 h, the conversion rate is detected. The immobilized enzyme is separated after each reaction ends and reused in the next reaction. The number of reuse is checked. Test results are shown in Table 15.

**Table 15**

| CFP-PVA-encapsulated enzyme | Main enzyme:coenzyme | Conversion rate (%) | Number of cycles |
|---|---|---|---|
| ERED-Sc | -/- | > 94% | 10 |
| ERED-Chr | -/- | > 90% | 11 |
| ERED-Sc+FDH | 5:1 | > 90% | 11 |
| | 2:1 | > 90% | 12 |
| | 1:1 | > 90% | 10 |
| | 1:2 | > 90% | 10 |
| | 1:5 | > 90% | 10 |
| | 1:10 | > 90% | 10 |
| | 1:12 | > 90% | 8 |
| ERED-Sc+GDH | 5:1 | > 90% | 13 |
| | 2:1 | 90% | 13 |
| | 1:1 | 90% | 11 |
| | 1:2 | 90% | 10 |
| ERED-Chr+GDH | 5:1 | > 90% | 11 |
| | 2:1 | > 90% | 11 |
| | 1:1 | > 90% | 10 |
| | 1:2 | > 90% | 10 |
| | 1:5 | > 90% | 9 |
| | 1:10 | > 90% | 7 |

### Embodiment 13

Imine reductase is immobilized by PVA-CFP membrane.

The immobilization method is the same as that in Embodiment 10, except that the PVA-encapsulated enzyme is changed to imine reductase IRED-Str or IRED-Bc, or may be a mixed enzyme of the alkene reductase and coenzyme glucose dehydrogenase GDH thereof or ammonium formate dehydrogenase FDH, and a ratio of the two enzymes is ERED:GDH (or FDH)=4:1.

Activity and stability are tested.

A reaction model used in translational research:

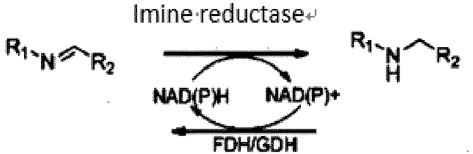

R1 and R2 in the above reaction formula may be independently selected from H, substituted or unsubstituted alkyl, substituted or unsubstituted cycloalkyl, substituted or unsubstituted aryl, substituted or unsubstituted aralkyl, substituted or unsubstituted heterocyclyl, or substituted or unsubstituted heterocycloalkyl. Alternatively, a fused aromatic ring is formed by R1 and R2 and a heterocyclic ring or an aromatic ring connected to R1 and R2.

The activity of the PVA-CFP membrane immobilized enzyme of IRED is detected by following substrate 8 by means of the following method.

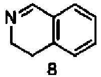

2 mLof a 0.1M PB buffer solution (pH 7.0-8.0) is put in the 10 ml reaction flask, and then 100 mg of the substrate 8, 10 mg of NAD(P)⁺, 60 mg of ammonium formate, 10 mg of FDH, and the PVA-CFP membrane immobilized enzyme containing 40 mg of IRED are added. After the reaction is performed at 30°C for 16 h, the conversion rate is detected. The immobilized enzyme is separated after each reaction ends and reused in the next reaction. The number of reuse is checked.

The activity of the PVA-CFP membrane immobilized enzyme of IRED and FDH is detected by following substrate 8 by means of the following method.

2 mL of the 0.1M PB buffer solution (pH 7.0-8.0) is put in the 10 ml reaction flask, and 100 mg of the substrate 8 is added; then 10 mg of NAD(P)⁺ and 60 mg of ammonium formate are added; and then, the PVA-CFP membrane immobilized enzyme containing 50 mg of a mixed enzyme of IRED and FDH is added. After the reaction is performed at 30°C for 16 h, the conversion rate is detected. The immobilized enzyme is separated after each reaction ends and reused in the next reaction. The number of reuse is checked.

The activity of the PVA-CFP membrane immobilized enzyme of IRED and GDH is detected by means of the following method.

3 mL of the 0.1M PB buffer solution (pH 7.0-8.0) is put in the 10 ml reaction flask, and 100 mg of the substrate 8 is added subsequently; then 10 mg of NAD(P)⁺, 100 mg of glucose, and the PVA-CFP membrane immobilized enzyme containing 50 mg of a mixed enzyme of IRED and GDH are added. After the reaction is performed at 30°C for 16 h, a conversion test is performed. Test results are shown in Table 16.

**Table 16**

| CFP-PVA-encapsulated enzyme | Conversion rate (%) | Number of cycles |
|---|---|---|
| IRED-Str | > 90% | 6 |
| IRED-Bc | > 90% | 7 |
| IRED-Str+FDH | > 90% | 5 |
| IRED-Str+GDH | > 90% | 6 |
| IRED-Bc+FDH | > 90% | 4 |
| IRED-Bc+GDH | > 90% | 6 |

### Embodiment 14

Nitrilase is immobilized by PVA-CFP membrane.

The immobilization method is the same as that in Embodiment 10, except that the PVA-encapsulated enzyme is changed to an enzyme solution (an enzyme concentration being 0.1 g/mL) of nitrilase NIT-An or NIT-Nc or cross-linked enzyme aggregate suspension liquid (0.5 g/mL) of NIT-An or NIT-Nc.

Activity and stability are tested.

A reaction model used in translational research:

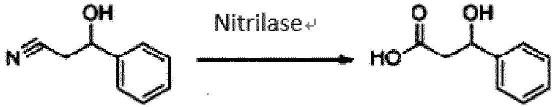

The activity of the PVA-CFP membrane immobilized enzyme of nitrilase is detected by following substrate 9 by means of the following method.

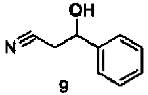

2 mL of the 0.1M PB buffer solution (pH 7.0-8.0) is added to the 10 ml reaction flask, and 100 mg of the substrate 9 is added; and then, the PVA-CFP membrane immobilized enzyme containing 20mg of NIT is added. After the reaction is performed at 30°C for 16 h, the conversion rate is detected. The immobilized enzyme is separated after each reaction ends and reused in the next reaction. The number of reuse is checked.

Test results are shown in Table 17.

**Table 17**

| CFP-PVA-encapsulated enzyme | Conversion rate (%) | Number of cycles |
|---|---|---|
| NIT-An | > 98% | 5 |
| NIT-Nc | > 98% | 4 |
| NIT-An cross-linked enzyme aggregate | > 98% | 8 |
| NIT-Nc cross-linked enzyme aggregate | > 98% | 8 |

### Embodiment 15

Ammonia lyase is immobilized by PVA-CFP membrane.

The immobilization method is the same as that in Embodiment 10, except that the PVA-encapsulated enzyme is changed to ammonia lyase PAL-An or PAL-Ss.

Activity and stability are tested.

A reaction model used in translational research:

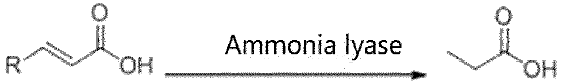

The activity of the PVA-CFP membrane immobilized enzyme of ammonia lyase PAL is detected by following substrate 10 by means of the following method.

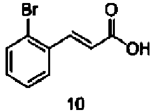

8 mL of a 4M ammonium carbamate aqueous solution (pH 9.0-9.5) is added to the 10 ml reaction flask, and 100 mg of the substrate 10 is added; and then, the PVA-CFP membrane immobilized enzyme containing 40mg of NIT is added. After the reaction is performed at 30°C for 16 h, the conversion rate is detected. The immobilized enzyme is separated after each reaction ends and reused in the next reaction. The number of reuse is checked.

Test results are shown in Table 18.

**Table 18**

| CFP-PVA-encapsulated enzyme | Conversion rate (%) | Number of cycles |
|---|---|---|
| PAL-An | > 98% | 18 |
| PAL-Ss | > 98% | 17 |

### Embodiment 16

Amino acid dehydrogenase is immobilized by PVA-CFP membrane.

The immobilization method is the same as that in Embodiment 10, except that the PVA-encapsulated enzyme is changed to amino acid dehydrogenase AADH-Bc or AADH-Bs, or may be a mixed enzyme of the amino acid dehydrogenase and coenzyme glucose dehydrogenase GDH thereof or ammonium formate dehydrogenase FDH, and a ratio of the two enzymes is AADH:GDH(or FDH)=4:1.

Activity and stability are tested.

A reaction model used in translational research:

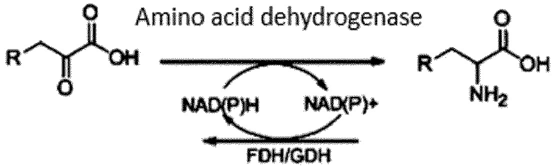

R is substituted or unsubstituted aryl.

The activity of the PVA-CFP membrane immobilized enzyme of AADH is detected by following substrate 11 or 12 by means of the following method.

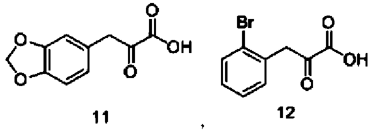

5 mL of a 0.1M Tris-CI buffer solution (pH 8.0-9.0) is added to the 10 ml reaction flask, 100 mg of the substrate 11 or 12 and 108 mg of ammonium chloride are added, and the pH is adjusted to 7.5-8.0; then 10 mg of NAD⁺, 150 mg of glucose and 10 mg of GDH are added; and finally, the PVA-CFP membrane immobilized enzyme containing 20 mg of AADH is added. After the reaction is performed at 30°C for 16 h, the conversion rate is detected. The immobilized enzyme is separated after each reaction ends and reused in the next reaction. The number of reuse is checked.

The activity of a co-immobilized enzyme of AADH and FDH is detected by means of the following method.

5 mL of the 0.1M Tris-CI buffer solution (pH 8.0-9.0) is added to the 10 ml reaction flask, 100 mg of the substrate 11 or 12 and 108 mg of ammonium chloride are added, and the pH is adjusted to 7.5-8.0; and then 10 mg of NAD⁺, 80 mg of ammonium formate, and the PVA-CFP membrane immobilized enzyme entrapped with 50 mg of the mixed enzyme of AADH and FDH are added. After the reaction is performed at 30°C for 16 h, the conversion rate is detected. The immobilized enzyme is separated after each reaction ends and reused in the next reaction. The number of reuse is checked.

The activity of the PVA-CFP membrane immobilized enzyme of AADH and GDH is detected by means of the following method.

5 mL of the 0.1M Tris-CI buffer solution (pH 8.0-9.0) is added to the 10 ml reaction flask, 100 mg of the substrate 11 or 12 and 108 mg of ammonium chloride are added, and the pH is adjusted to 7.5-8.0; and then 10 mg of NAD⁺, 150 mg of glucose and the PVA-CFP membrane immobilized enzyme entrapped with 50 mg of the mixed enzyme of AADH and GDH are added. After the reaction is performed at 30°C for 16 h, the conversion rate is detected. The immobilized enzyme is separated after each reaction ends and reused in the next reaction. The number of reuse is checked. Test results are shown in Table 19.

**Table 19**

| CFP-PVA-encapsulated enzyme | Conversion rate (%) | Number of cycles |
|---|---|---|
| AADH-Bc | > 95% | 7 |
| AADH-Bs | > 95% | 9 |
| AADH-Bc+FDH | > 95% | 6 |
| AADH-Bc+GDH | > 95% | 8 |
| AADH-Bs+FDH | > 95% | 8 |
| AADH-Bs+GDH | > 95% | 7 |

It may be seen from the above description that, in the above embodiments of the present disclosure, the following technical effects are realized.

In this disclosure, by means of using the PVA porous membrane as the carrier to immobilize the enzyme in an entrapment manner, the entrapment and immobilization process is simple, conditions are mild, and desirable immobilization effect is achieved on either purified enzymes or crude enzymes. After entrapping and immobilizing the enzyme in the PVA porous membrane, the enzyme is stable, and cannot be easily leached out during use. The porous structure of the PVA porous membrane used can better transmit reactants and products, and is suitable for use in continuous flow biochemical catalysis. Since entrapment and immobilization described above is mechanical immobilization, the porous membrane has wide applicability to enzymes. As the three-dimensional structured PVA porous membrane has the three-dimensional structure, the PVA porous membrane may have more specific surface areas, so that more entrapment sites can be provided. Therefore, the loading amount of the enzymes is increasedon the basis of guaranteeing of high activity and stability of the enzymes.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure all fall within the scope of protection of the present disclosure.

## Claims

1. A PVA membrane immobilized enzyme, comprising a PVA porous membrane and an enzyme entrapped on the PVA porous membrane, wherein the PVA porous membrane is a three-dimensional structured PVA porous membrane; and the enzyme is any one selected from transaminase, D-lactate dehydrogenase, cyclohexanone monooxygenase, ketoreductase, alkene reductase, nitrilase, ammonia lyase, amino acid dehydrogenase, imine reductase, alcohol dehydrogenase, ammonium formate dehydrogenase, glucose 1 -dehydrogenase or mutants thereof.

2. The PVA membrane immobilized enzyme according to claim 1, wherein the three-dimensional structured PVA porous membrane has a three-dimensional structures that are formed by protrusions or grooves; and preferably, the enzyme is a free enzyme or a cross-linked enzyme aggregate.

3. The PVA membrane immobilized enzyme according to claim 1 or 2, wherein the transaminase is a transaminase derived from *ChromobacteriumviolaceumDSM30191,* or a transaminase derived from *Arthrobacter citreus,* or a transaminase derived from *B.thuringiensis;* the ketoreductase is a ketoreductase derived from *Acetobacter sp. CCTCC M209061* or a ketoreductase derived from *Candida macedoniensis AKU4588;* the cyclohexanone monooxygenase is a cyclohexanone monooxygenase derived from *Rhodococcussp. Phil,* or a cyclohexanone monooxygenase derived from *Brachymonaspetroleovorans,* or a cyclohexanone monooxygenase derived from *Rhodococcus ruber-SD1;* the ammonia lyase is an ammonia lyase derived from *Aspergillus niger CBS 513.88or* anammonia lyase derived from *Solenostemonscutellarioides;* the alkene reductase is an alkene reductase derived from *Saccharomyces cerevisiae* or analkene reductase derived from *Chryseobacteriumsp. CA49;* the imine reductase is an imine reductase derived from *Streptomyces* spot animine reductase derived from *Bacillus cereus;* the amino acid dehydrogenase is an amino acid dehydrogenase derived from *Bacillus cereusor* anamino acid dehydrogenase derived from *Bacillus sphaericus;* the nitrilase is a nitrilase derived from *Aspergillus niger CBS 513.88or* anitrilase derived from *Neurospora crassa OR74A;* preferably, the transaminase derived from *Chromobacteriumviolaceum DSM30191* has an amino acid sequence shown in SEQ ID NO.1, and an amino acid sequence of a mutant of the transaminase is an amino acid sequence that is obtained by the mutation of the amino acid sequence shown in SEQ ID NO.1, wherein the mutation comprises at least one of the following mutation sites: 7th site, 47th site, 90th site, 95th site, 297th site, 304th site, 380th site, 405th site or 416th site, and threonine at the 7th site is mutated to cysteine, serine at the 47th site is mutated to the cysteine, lysine at the 90th site is mutated to glycine, alanine at the 95th site is mutated to proline, isoleucine at the 297th site is mutated to leucine, the lysine at the 304th site is mutated to aspartic acid, glutamine at the 380th site is mutated to the leucine, arginine at the 405th site is mutated to the glutamate, and the arginine at the 416th site is mutated to threonine, or the amino acid sequence of the mutant of the transaminase has the mutation site in the amino acid sequence that is obtained by means of mutation, and is of more than 80% identity with the amino acid sequence that is obtained by means of mutation; preferably, the transaminase derived from *Arthrobacter citreus* has an amino acid sequence shown in SEQ ID NO.2, and an amino acid sequence of a mutant of the transaminase is an amino acid sequence that is obtained by a mutation of the amino acid sequence shown in SEQ ID NO.2, wherein the mutation comprises at least one of the following mutation sites: 3rd site, 5th site, 60th site, 164th site, 171st site, 178th site, 180th site, 186th site, 187th site, 252nd site, 370th site, 384th site, 389th site, 404th site, 411th site, 423rd site, or 424th site, and the leucine at the 3rd site is mutated to the serine, valine at the 5th site is mutated to the serine, the cysteine at the 60th site is mutated to tyrosine, phenylalanine at the 164th site is mutated to the leucine, the glutamate at the 171st site is mutated to the aspartic acid, the alanine at the 178th site is mutated to the leucine, the isoleucine at the 180th site is mutated to the valine, the serine at the 186th site is mutated to glycine, the serine at the 187th site is mutated to the alanine, the valine at the 252nd site is mutated to the isoleucine, the leucine at the 370th site is mutated to the alanine, tyrosine at the 384th site is mutated to the phenylalanine, the isoleucine at the 389th site is mutated to the phenylalanine, the leucine at the 404th site is mutated to the glutamine, the glycine at the 411th site is mutated to the aspartic acid, methionine at the 423rd site is mutated to the lysine, and the glutamate at the 424th site is mutated to the glutamine, or the amino acid sequence of the mutant of the transaminase has the mutation site in the amino acid sequence that is obtained by means of mutation, and is of more than 80% identity with the amino acid sequence that is obtained by means of mutation; preferably, the ketoreductase derived from *Acetobacter sp. CCTCC M209061* has an amino acid sequence shown in SEQ ID NO.3, and an amino acid sequence of a mutant of the ketoreductase is an amino acid sequence that is obtained by a mutation of the amino acid sequence shown in SEQ ID NO.3, wherein the mutation comprises at least one of the following mutation sites: 94th site, 144th site, or 156th site, and the alanine at the 94th site is mutated to asparagine, the glutamate at the 144th site is mutated to the serine, and the asparagine at the 156th site is mutated to the threonine or the valine, or the amino acid sequence of the mutant of the ketoreductase has the mutation site in the amino acid sequence that is obtained by means of mutation, and is of more than 80% identity with the amino acid sequence that is obtained by means of mutation; preferably, the cyclohexanone monooxygenase derived *fromRhodococcussp. Phil* has an amino acid sequence shown in SEQ ID NO.4, and an amino acid sequence of a mutant of the cyclohexanone monooxygenase is an amino acid sequence that is obtained by a mutation of the amino acid sequence shown in SEQ ID NO.4, wherein the mutation comprises at least one of the following mutation sites: 280th site, 435th site, 436th site, 438th site, 411st site, 508th site, or 510th site, and the phenylalanine at the 280th site is mutated to the tyrosine, the phenylalanine at the 435th site is mutated to the asparagine, the phenylalanine at the 436th site is mutated to the serine, the leucine at the 438th site is mutated to the alanine, serine at the 411st site is mutated to the valine, and the leucine at the 510th site is mutated to the valine, or the amino acid sequence of the mutant of the cyclohexanone monooxygenase has the mutation site in the amino acid sequence that is obtained by means of mutation, and is of more than 80% identity with the amino acid sequence that is obtained by means of mutation; and preferably, the cyclohexanone monooxygenase derived from *Rhodococcus ruber-SD1* has an amino acid sequence shown in SEQ ID NO.5, and an amino acid sequence of the mutant of the cyclohexanone monooxygenase is an amino acid sequence that is obtained by a mutation of the amino acid sequence shown in SEQ ID NO.5, wherein the mutation comprises at least one of the following mutation sites: 45th site, 190th site, 249th site, 257th site, 393rd site, 504th site, or 559th site, and methionine at the 45th site is mutated to the threonine, proline at the 190th site is mutated to the leucine, the cysteine at the 249th site is mutated to the valine, the cysteine at the 257th site is mutated is the alanine, the cysteine at the 393rd site is mutated to the valine, the proline at the 504th site is mutated to the valine, and the tyrosine at the 559th site is mutated to the methionine, or the amino acid sequence of the mutant of the cyclohexanone monooxygenase has the mutation site in the amino acid sequence that is obtained by means of mutation, and is of more than 80% identity with the amino acid sequence that is obtained by means of mutation.

4. The PVA membrane immobilized enzyme according to claim 1, further comprising a coenzyme and a cofactor of each enzyme, wherein the coenzyme and the cofactor are entrapped on the PVA porous membrane.

5. The PVA membrane immobilized enzyme according to any one of claims 1 to 4, wherein the PVA porous membrane further has polyethylene glycol and/or polyethyleneimine; preferably, a molecular weight of the polyethylene glycol is PEG400-PEG 6000, and a molecular weight of the polyethyleneimine is 3KDa-70KDa; and preferably, a mass ratio of the polyethylene glycol to the porous PVA is 5:4-75:4, and a mass ratio of the polyethyleneimine to the porous PVA is 1:12-1:240.

6. The PVA membrane immobilized enzyme according to claim 1, wherein the enzyme is a crude enzyme.

7. The PVA membrane immobilized enzyme according to claim 1 or 6, wherein the loading amount of the enzyme is 0.05~0.4 g of free enzyme/cm² membrane or 0.03~0.06 g of dry cross-linked enzyme aggregate/cm² membrane.

8. A method for preparing the PVA membrane immobilized enzyme according to anyone of claims 1 to 7, comprising:
S1, mixing a raw material comprising an enzyme an a PVA solution for scheduled time, to obtain a mixed system;
S2, adding the mixed system to a mold, and drying the mixed system to obtain a membrane-entrapped enzyme, wherein the mold is a three-dimensional structured mold so as to form a three-dimensional structured PVA porous membrane; and
S3, using a phosphate buffer solution to soak and wash the membrane-entrapped enzyme, and then obtaining the PVA membrane immobilized enzyme, wherein preferably, a pH value of the mixed system is 6.0-6.5.

9. The preparation method according to claim 8, wherein, S1 comprises:
preparing suspension liquid or an enzyme solution of the enzyme, wherein the enzyme in the suspension liquid is a cross-linked enzyme aggregate, and the enzyme in the enzyme solution is a free enzyme without cells; and
mixing the suspension liquid or the enzyme solution with the PVA solution for the scheduled time, to obtain the mixed system, wherein preferably, the scheduled time is 10-60 min; a PVA molecular weight of the PVA solution is 20KDa-200KDa; preferably, the content of PVA in the PVA solution is 10-50 g/100 mL; preferably, acetic acid, methanol and sulfuric acid are dispersed in the PVA solution; preferably, a pH value of the PVA solution is 5.5-6.5; preferably, a ratio of the enzyme to the PVA solution is 1-50 g/100 mL; preferably, the suspension liquid or the enzyme solution further contains the phosphate buffer solution, an optional cofactor and an optional coenzyme; and preferably, a weight ratio of the coenzyme to the enzyme is 10:1-1 :10.

10. The preparation method according to claim 8, wherein, S1 comprises:
mixing a PVA aqueous solution and cross-linked enzyme particles to form the mixed system, wherein preferably, a ratio of the cross-linked enzyme particle to the PVA aqueous solution is 1-50 g/100 mL; preferably, the scheduled time is 10-60 min; and preferably, the cross-linked enzyme particle comprises the enzyme, an optional cofactor and an optional coenzyme.

11. The preparation method according to claim 8, wherein, S1 comprises:
mixing a PVA aqueous solution and a modifier solution for a first scheduled time, to form a second mixed system; and
mixing the second mixed system and an enzyme system for a second scheduled time, to form the mixed system, wherein preferably, a concentration of the PVA aqueous solution is 5-30 g/100 mL; preferably, the modifier solution comprises a polyethylene glycol aqueous solution in which the cofactors are dispersed and/or a polyethyleneimine aqueous solution in which the cofactors are dispersed; preferably, a molecular weight of the polyethylene glycol is PEG400-PEG6000; preferably, a concentration of the polyethylene glycol in the mixed system is 3-10 g/100 mL; preferably, a molecular weight of the polyethyleneimine is 3KDa-70KDa, and more preferably, the molecular weight of the polyethyleneimine is 3KDa-50KDa; preferably, a concentration of the polyethyleneimine in the mixed system is 0.1-1 g/100 mL, and more preferably, the concentration of the polyethyleneimine in the mixed system is 0.1-0.3 g/100 mL; preferably, the enzyme system comprises the enzyme, the optional cofactor, the optional coenzyme and the phosphate buffer solution; preferably, the enzyme is a free enzyme or a cross-linked enzyme aggregate without cells; preferably, a concentration of the cofactor in the enzyme system is 1-20 mg/mL; preferably, a weight ratio of the coenzyme in the enzyme system to the enzyme is 10:1-1:10; and preferably, a ratio of the enzyme to the PVA solution is 1-50 g/100 m L.

12. The preparation method according to any one of claims 8 to 11, wherein, S2 comprises:
placing the mixed system in a mold for athird scheduled time, and then adding a dehydration accelerator to the mold for drying, wherein the dehydration accelerator isany one or more selected from a group consisting of acetonitrile, ethanol and acetone; preferably, a volume ratio of the dehydration accelerator to the mixed system is 1:10-5:1; preferably, the third scheduled time is 2-4 h; and preferably, the mold is the three-dimensional structured mold, and the three-dimensional structured mold has protrusions or grooves.

13. The preparation method according to any one of claims 8 to 11, wherein, S3 comprises:
soaking the membrane-entrapped enzyme in the phosphate buffer solution for 2-16 h, and then using the fresh phosphate buffer solution to wash the membrane-entrapped enzyme, so as to obtain the PVA membrane immobilized enzyme.
